# NEW EUROPEAN PATENT SPECIFICATION

(11) **EP 0 314 317 B2**
(45) Date of publication and mention of the opposition decision: **23.03.2005**
(45) Mention of the grant of the patent: 26.08.1998
(21) Application number: 88309194.4
(22) Date of filing: 03.10.1988
(51) Int. Cl.: C12N 15/00, C12P 21/02, A61K 38/10, G01N 33/566

(54) **Adheson variants, nucleic acid encoding them and compositions comprising them**
Adhäsionsvarianten, dafür kodierende Nukleinsäure und diese enthaltende Zusammensetzungen
Variantes d'adhésions, acide nucléique les codant ainsi que compositions les contenant

(30) Priority: 02.10.1987 US 104329; 28.09.1988 US 250785
(43) Date of publication of application: 03.05.1989
(73) Proprietor: Genentech Inc., South San Francisco, CA 94080-4990 (US)
(72) Inventor: Capon, Daniel J., San Mateo California 94402 (US); Gregory, Timothy J., Hillsborough California 94010 (US)
(74) Representative: Armitage, Ian Michael

(56) References cited:
- WO-A-88/01304
- MADDON P J ET AL: "THE ISOLATION AND NUCLEOTIDE SEQUENCE OF A CDNA ENCODING THE T CELL SURFACE PROTEIN T4: A NEW MEMBER OF THE IMMUNOGLOBULIN GENE FAMILY" CELL, CELL PRESS, CAMBRIDGE, NA, US, vol. 42, no. 1, 1 August 1985 (1985-08-01), pages 93-104, XP002051712 ISSN: 0092-8674
- CLARK S J ET AL: "PEPTIDE AND NUCLEOTIDE SEQUENCES OF RAT CD4 (W3/25) ANTIGEN: EVIDENCE FOR DERIVATION FROM A STRUCTURE WITH FOUR IMMUNOGLOBULIN-RELATED DOMAINS" PROCEEDINGS OF THE NATIONAL ACADEMY OF SCIENCES OF USA, NATIONAL ACADEMY OF SCIENCE. WASHINGTON, US, vol. 84, 1 March 1987 (1987-03-01), pages 1649-1653, XP002051713 ISSN: 0027-8424
- D.H.SMITH ET AL : "Blocking of HIV-1 infectivity by a soluble, secreted form of the CD4 antigen" SCIENCE, vol. 238, 18 December 1987 (1987-12-18), pages 1704-1707, XP002964490

## Description

This application relates to compositions for antiviral or immunomodulatory therapy. In particular, it relates to compositions useful in the treatment of Human Immunodeficiency Virus (HIV) infections.

The primary immunologic abnormality resulting from infection by HIV is the progressive depletion and functional impairment of T lymphocytes expressing the CD4 cell surface glycoprotein (H. Lane et al., Ann. Rev. Immunol. 3:477 [1985]). CD4 is a non-polymorphic glycoprotein with homology to the immunoglobulin gene superfamily (P. Maddon et al., Cell 42:93 [1985]). Together with the CD8 surface antigen, CD4 defines two distinct subsets of mature peripheral T cells (E. Reinherz et al., Cell 19:821 [1980]), which are distinguished by their ability to interact with nominal antigen targets in the context of class I and class II major histocompatibility complex (MHC) antigens, respectively (S. Swain, Proc. Natl. Acad. Sci. 78:7101 [1981]; E. Engleman et al., J. Immunol. 127:2124 [1981]; H. Spitz et al., J. Immunol. 129:1563 [1982]; W. Biddison et al., J. Exp. Med. 156:1065 [1982]; and D. Wilde et al., J. Immunol. 131:2178 [1983]). For the most part, CD4 T cells display the helper/inducer T cell phenotype (E. Reinherz, supra), although CD4 T cells characterized as cytotoxic/suppressor T cells have also been identified (Y Thomas et al., J. Exp. Med. 154:459 [1981]; J; S. Meuer et al., Proc. Natl. Acad. Sci. USA 79:4395[1982]; and A. Krensky et al., Proc. Natl. Acad. Sci. USA 79:2365 [1982]). The loss of CD4 helper/inducer T cell function probably underlies the profound defects in cellular and humoral immunity leading to the opportunistic infections and malignancies characteristic of the acquired immunodeficiency syndrome (AIDS) (H. Lane supra).

Studies of HIV-1 infection of fractionated CD4 and CD8 T cells from normal donors and AIDS patients have revealed that depletion of CD4 T cells results from the ability of HIV-I to selectively infect, replicate in, and ultimately destroy this T lymphocyte subset (D. Klatzmann et al., Science 225:59 [1984]). The possibility that CD4 itself is an essential component of the cellular receptor for HIV-I was first indicated by the observation that monoclonal antibodies directed against CD4 block HIV-I infection and syncytia induction (A. Dalgleish et al., Nature [London] 312:767 [1984]; J. Mc-Dougal et al., J. Immunol. 135:3151 [1985]). This hypothesis has been confirmed by the demonstration that a molecular complex forms between CD4 and gp120, the major envelope glycoprotein of HIV-I (J. McDougal et al., Science 231: 382 [1986]; and the finding that HIV-I tropism can be conferred upon ordinarily non-permissive human cells following the stable expression of a CD4 cDNA (P. Maddon et al., Cell 47:333 [1986]). Furthermore, the neurotropic properties of HIV-I, reflected by a high incidence of central nervous system dysfunction in HIV-I infected individuals (W. Snider et al., Ann. Neurol. 14:403 [1983]), and the ability to detect HIV-I in the brain tissue and cerebrospinal fluid of AIDS patients (G. Shaw et al., Science 227:177 [1985]; L. Epstein, AIDS Res. 1:447 [1985]; S. Koenig, Science 233:1089 [1986]; D. Ho et al., N. Engl. J. Med. 313:1498 [1985]; J. Levy et al., Lancet II:586 [1985]), appears to have its explanation in the expression of CD4 in cells of neuronal, glial and monocyte/macrophage origin (P. Maddon, Cell 47:444 [1986]; I. Funke et al., J. Exp. Med. 165:1230 [1986]; B. Tourvieille et al., Science 234:610 [1986]).

In addition to determining the susceptibility to HIV-I infection, the manifestation of cytopathic effects in the infected host cell appears to involve CD4. Antibody to CD4 was found to inhibit the fusion of uninfected CD4 T cells with HIV-I infected cells in vitro; moreover, the giant multinucleated cells produced by this event die shortly after being formed resulting in the depletion of the population of CD4 cells (J. Lifson et al., Science 232:1123 [1986]). Formation of syncytia also requires gp120 expression, and can be elicited by coculturing CD4-positive cell lines with cell lines expressing the HIV-I env gene in the absence of other viral structural or regulatory proteins (J. Sodroski et al., Nature 322:470 [1986]; J. Lifson et al., Nature 323:725 [1986]). Thus, in mediating both the initial infection by HIV-I as well as eventual cell death, the interaction between gp120 and CD4 constitutes one of several critical entry points in the viral life cycle amenable to therapeutic intervention (H. Mitsuya et al., Nature 325:773 [1987]).

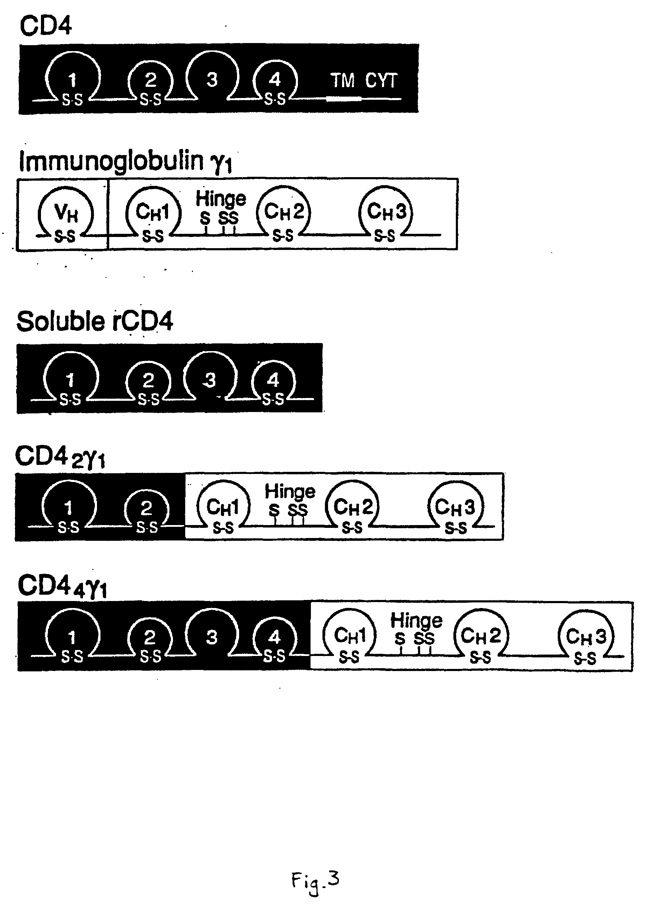

The known sequence of the CD4 precursor predicts a hydrophobic signal peptide, an extracellular region of approximately 370 amino acids, a highly hydrophobic stretch with significant identity to the membrane-spanning domain of the class II MHC beta chain, and a highly charged intracellular sequence of 40 residues (P. Madden, Cell 42:93 [1985]). The extracellular domain of CD4 consists of four contiguous regions each having amino acid and structural similarity to the variable and joining (V-J) domains of immunoglobulin light chains as well as related regions in other members of the immunoglobulin gene superfamily (a subclass of which are defined herein by the coined term "adhesons". These structurally similar regions of CD4 are termed the V₁J₁, V₂J₂, V₃J₃ and V₄J₄ domains (denominated 1-4 in Fig. 3).

A successful strategy in the development of drugs for the treatment of many receptor mediated abnormalities has been the identification of antagonists which block binding of the natural ligand. Since the CD4 adheson ordinarily binds to the recognition sites of the HIV envelope it would appear to be a candidate for therapeutically sequestering these HIV sites, thereby blocking viral infectivity. However, full length CD4 and other adhesons are cell membrane proteins which are anchored in the lipid bilayer of lymphocytes. The presence of membrane components will be undesirable from the standpoint of manufacturing and purification. In addition, since adhesons are normally present only on cell surfaces, it would be desirable to produce adhesons in a form which is more stable in the circulation. Additionally, even truncated, soluble CD4 adheson (generally referred to as CD4T) may not be optimally effective as a therapeutic since it possesses a relatively short biological half-life, binds to HIV no better than cell surface CD4, may not cross the placental or other biological barriers and since it merely sequesters the HIV recognition sites without in itself bearing an infected-cell killing or virus killing functionality.

Accordingly, it is desirable to produce soluble, secreted adhesons. It is also desirable to produce CD4 derivatives useful in the treatment of AIDS and related conditions, in a manner essentially unaffected by the extreme degree of genetic variation observed among various HIV-I isolates and their respective env polypeptides (J.Coffin, Cell 46:1 [1986]). It is further desirable to prepare adhesons fused to other polypeptides in order to provide molecules with novel functionalities such as those described above for therapeutic use, or diagnostic reagents for the *in vitro* assay of adhesons or their ligands. In particular, it is desirable to prepare molecules for directing toxins or effector molecules (for example the Fc domain of immunoglobulin) to cells bearing receptors for the adhesons, e.g. HIV gp120 in the case of CD4, and for use in facilitating purification of the adhesons. It is further desirable to provide stable, highly purified adheson preparations.

The present invention provides nucleic acid encoding an amino acid sequence variant of an adheson (CD4), in particular a variant in which the trans-membrane domain is modified so that it is no longer capable of becoming lodged in the cell membrane, i.e. inactivated, for example, by deletion or substitution.

Variant adhesons are produced by a method comprising (a) transforming a host cell with nucleic acid encoding an amino acid sequence variant of an adheson, (b) culturing the host cell and (c) recovering the variant adheson from the host cell culture media.

In another embodiment the invention provides an adheson variant selected from the group consisting of (a) a CD4 adheson amino acid sequence variant having an inactivated transmembrane domain and (b) a polypeptide comprising an adheson extracellular domain fused to the sequence of a polypeptide which is extraneous to the adheson, this latter for CD4 extracellular domain being for example selected from a cytotoxin, an immunogen or an immunoglobulin constant domain, and for other adheson extracellular domains being an immunoglobulin constant region.

In a preferred embodiment a polypeptide comprising a gp120 binding domain of the CD4 adheson is fused at its C-terminus to an immunoglobulin constant domain, or is linked to a cytotoxic polypeptide such as ricin.

The CD4 adheson variants provided herein are purified and formulated in pharmacologically acceptable vehicles for administration to patients in need of antiviral or immunomodulatory therapy, in particular patients infected with HIV.

### Brief Description of the Drawings

Figs. 1a-1c depict the amino,acid and nucleotide sequence of a secreted form of the CD4 adheson (CD4T). Other forms of CD4T terminate at Ser 366 or Pro 368. The signal processing site is designated with an arrow.

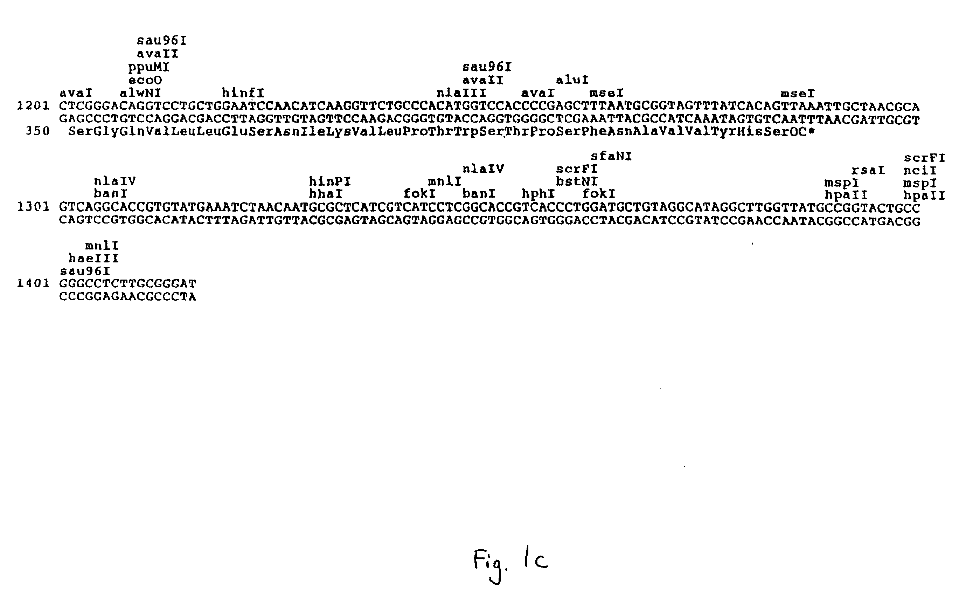

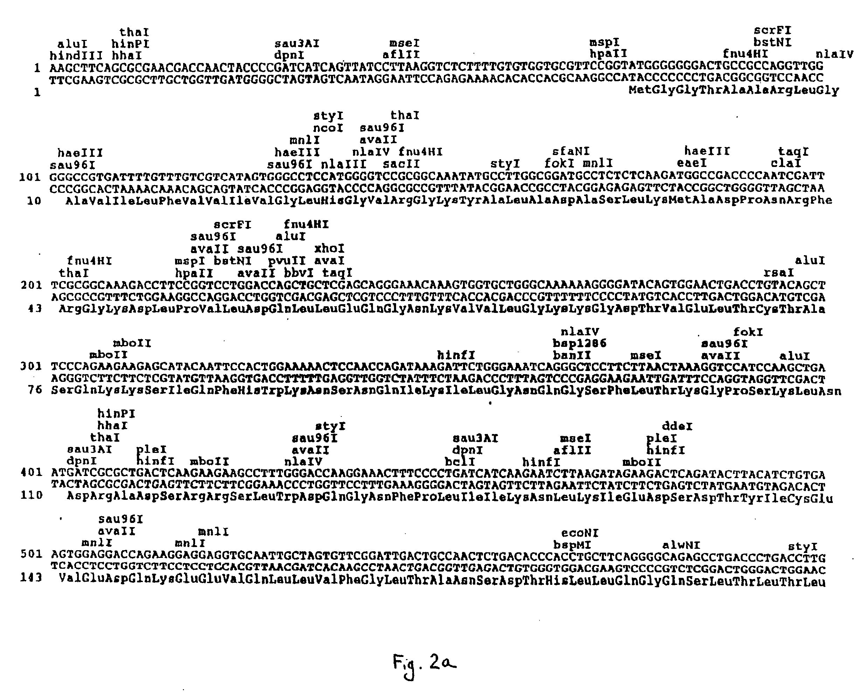

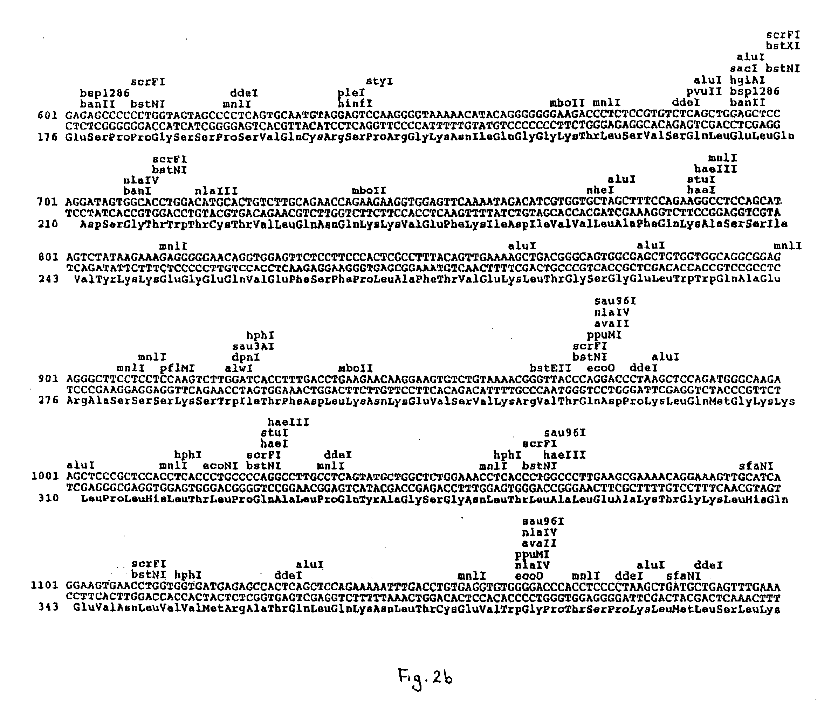

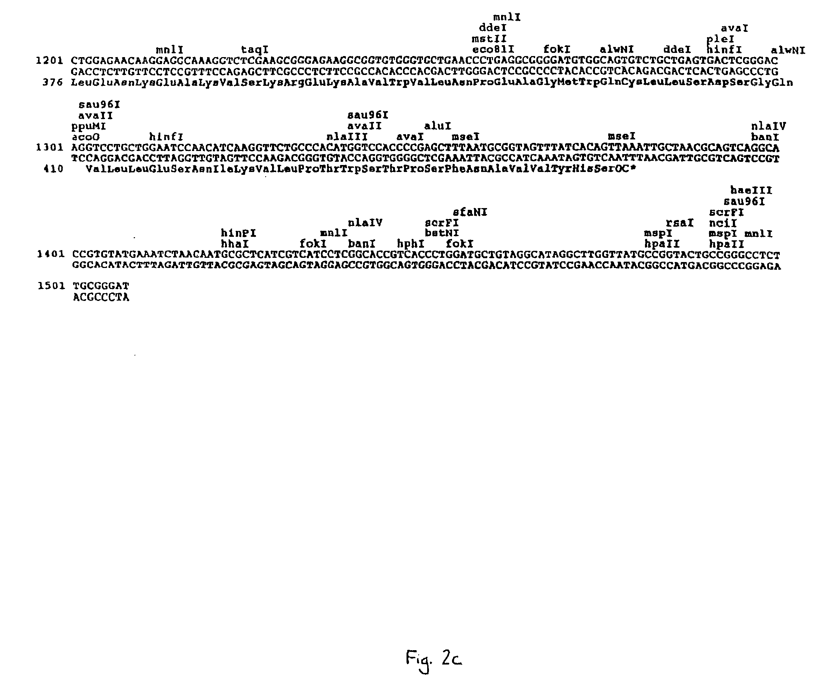

Figs. 2a-2c depict the amino acid and nucleotide sequence of a fusion of the herpes gD leader and N-terminal 27 residues to the putative mature N-terminus of CD4T.
Fig. 3 depicts the structural elements of the native and soluble CD4 antigen, the native immunoglobulin G heavy (γ) chain and two exemplary heavy chain-CD4 chimeras.

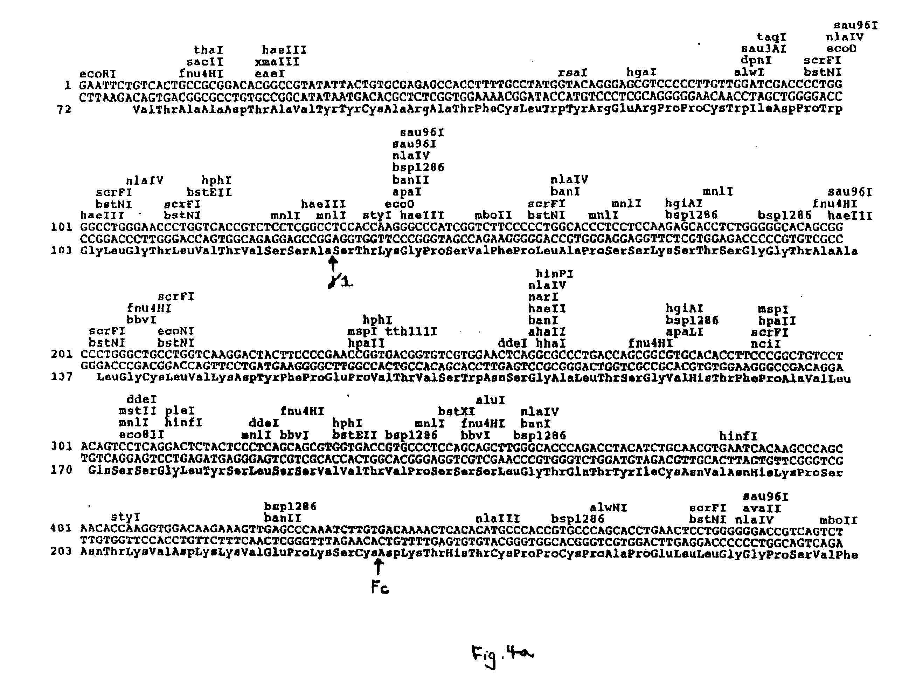

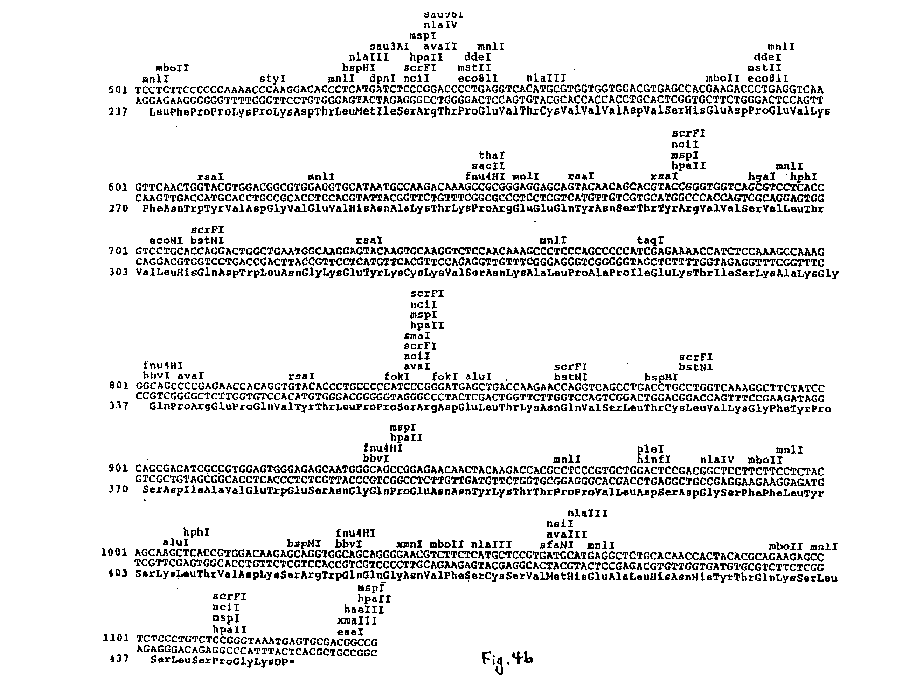

Figs. 4a-4b are a map of the linkered immunoglobulin γ chain fragment employed in the preparation of CD4 fusions. Insert sites are designated γ1 and Fc.
Fig. 5 is a map of a human light chain fragment useful for CD4 fusions at the arrow flanked by VₖJₖ (light variable and joining) and Cₖ (light constant).

### Detailed Description

Adhesons are cell surface polypeptides having an extracellular domain which is of a member of the immunoglobulin gene superfamily, excluding, however, highly polymorphic members of this superfamily selected from the group of class I and class II major histocompatibility antigens, immunoglobulins and T-cell receptor α, β, γ and δ chains. Examples of adhesons include CD2, CD4, CD8, CD28, the γ, δ and ε chains of CD3, OX-2, Thy-1, the intercellular or neural cell adhesion molecules (I-CAM or N-CAM), neurocytoplasmic protein (NCP-3), poly-Ig receptor, myelin-associated glycoprotein (MAG), high affinity IgE receptor, the major glycoprotein of peripheral myelin (Po), platelet derived growth factor receptor, colony stimulating factor-1 receptor, macrophage Fc receptor, Fc gamma receptors and carcinoembryonic antigen. Preferred adhesons are CD4, CD8 and high affinity IgE receptor.

The present invention in one aspect provides a process which comprises making an immunoglobulin heavy chain dimer in which the extracellular domain sequence of a cell surface member of the immunoglobulin gene superfamily, which superfamily member is not a class I or class II major histocompatibility antigen, an immunoglobulin, nor a T-cell receptor α, β, γ or δ chain, is substituted for the variable region of an immunoglobulin heavy chain, which dimer comprises a polypeptide comprising said extracellular domain fused to an immunoglobulin heavy chain constant region, and in which dimer said extracellular domain binds a ligand of said member of the immunoglobulin gene superfamily. Such an immunoglobulin heavy chain dimer may be divalent for said ligand.

In another aspect, the present invention provides a process which comprises making a polypeptide comprising an extracellular domain of CD4 fused to a polypeptide extraneous to CD4.

In another aspect, the present invention provides a process which comprises making a CD4 polypeptide amino acid sequence variant (a) having a transmembrane domain modified to be incapable of becoming lodged in the cell membrane, or (b) lacking a transmembrane domain.

Described herein are amino acid sequence variants of adhesons. Amino acid sequence variants of adhesons are prepared with various objectives in mind, including increasing the affinity of the adheson for its binding partner, facilitating the stability, purification and preparation of the adheson, increasing its plasma half life, improving therapeutic efficacy as described above in the background, introducing additional functionalities and lessening the severity or occurrence of side effects during therapeutic use of the adheson. Amino acid sequence variants of adhesons fall into one or a combination of the following classes: insertional, substitutional or deletional variants.

Insertional amino acid sequence variants are those in which one or more amino acid residues extraneous to the adheson are introduced into a predetermined site in the adheson including the C or N termini. Such variants are referred to as fusions of the adheson and a different polypeptide are produced. Such other polypeptides contain sequences other than those which are normally found in the adheson at the inserted position. Several groups of fusions are described. Immunologically active adheson fusions comprise an adheson and a polypeptide containing a non-adheson epitope. The non-adheson epitope is any immunologically competent polypeptide, i.e., any polypeptide which is capable of eliciting an immune response in the animal to which the fusion is to be administered or which is capable of being bound by an antibody raised against the non-adheson polypeptide. Typical non-adheson epitopes will be those which are borne by allergens, autoimmune epitopes, or other potent immunogens or antigens recognized by pre-existing antibodies in the fusion recipient, including bacterial polypeptides such as trpLE, beta-galactosidase, viral polypeptides such as herpes gD protein, and the like. Immunogenic fusions are produced by cross-linking in vitro or by recombinant cell culture transformed with DNA encoding an immunogenic polypeptide. It is preferable that the immunogenic fusion be one in which the immunogenic sequence is joined to or inserted into the adheson antigen or fragment thereof by a peptide bond(s). These products therefore consist of a linear polypeptide chain containing adheson epitopes and at least one epitope foreign to the adheson. The epitopes can be introduced anywhere within the adheson molecule or fragment thereof. Such fusions are conveniently made in recombinant host cells or by the use of bifunctional cross-linking agents. The use of a cross-linking agent to fuse the adheson to the immunogenic polypeptide is not as desirable as a linear fusion because the crosslinked products are not as easily synthesized in structurally homogeneous form.

These immunogenic insertions are particularly useful when formulated into a pharmacologically acceptable carrier and administered to a subject in order to raise antibodies against the adheson, which antibodies in turn are useful in diagnostics or in purification of adheson by immunoaffinity techniques known per se. Alternatively, in the purification of adhesons, binding partners for the fused non-adheson polypeptide, e.g. antibodies, receptors or ligands, are used to adsorb the fusion from impure admixtures, after which the fusion is eluted and, if desired, the adheson is recovered from the fusion, e.g. by enzymatic cleavage.

Other fusions, which may or may not also be immunologically active, include fusions of the adheson sequence with a signal sequence heterologous to the adheson, fusions of transmembrane-modified CD4 adhesons, for example, to polypeptides having enhanced plasma half life (ordinarily >about 20 hours) such as immunoglobulin chains or fragments thereof, and fusions with cytotoxic functionalities. Signal sequence fusions are employed in order to more expeditiously direct the secretion of the adheson. The heterologous signal replaces the native adheson signal, and when the resulting fusion is recognized, i.e. processed and cleaved by the host cell, the adheson is secreted. Signals are selected based on the intended host cell, and may include bacterial yeast, mammalian and viral sequences. The herpes gD glycoprotein signal is suitable for use in mammalian expression systems.

Plasma proteins which have enhanced plasma half-life longer than that of transmembrane modified CD4 include serum albumin, immunoglobulins, apolipoproteins, and transferrin. Preferably, the adheson-plasma protein fusion is not significantly immunogenic in the animal in which it is used and the plasma protein does not cause undesirable side effects in patients by virtue of its normal biological activity.

In a specific embodiment the adheson extracellular domain is conjugated with an immunoglobulin constant region sequence. The resulting products are referred to herein as immunoadhesons. Immunoglobulins and certain variants thereof are known and many have been prepared in recombinant cell culture. For example, see U.S. Patent 4,745,055; EP 256,654; Faulkner et al., Nature 298:286 (1982); EP 120,694; EP 125,023; Morrison, J. Immun. 123:793 (1979); Köhler et al., P.N.A.S. USA 77:2197 (1980); Raso et al., Cancer Res. 41:2073 (1981); Morrison et al., Ann. Rev. Im-, munol. 2:239 (1984); Morrison, Science 229:1202 (1985); Morrison et al., P.N.A.S. USA 81:6851 (1984); EP 255,694; EP 266,663; and WO 88/03559. Reassorted immunoglobulin chains also are known. See for example U.S. patent 4,444,878; WO 88/03565; and EP 68,763 and references cited therein.

Ordinarily, the domains of adhesons that are homologous to immunoglobulins and extracellular in their native environment are fused C-terminally to the N-terminus of the constant region of immunoglobulins in place of the variable region(s)-like thereof, retaining at least functionally active hinge, CH2 and CH3 domains of the constant region of an immunoglobulin heavy chain. This ordinarily is accomplished by constructing the appropriate DNA sequence and expressing it in recombinant cell culture. Immunoglobulins and other polypeptides having enhanced plasma half life are fused to the extracellular or ligand binding domains of other adhesons in the same fashion.

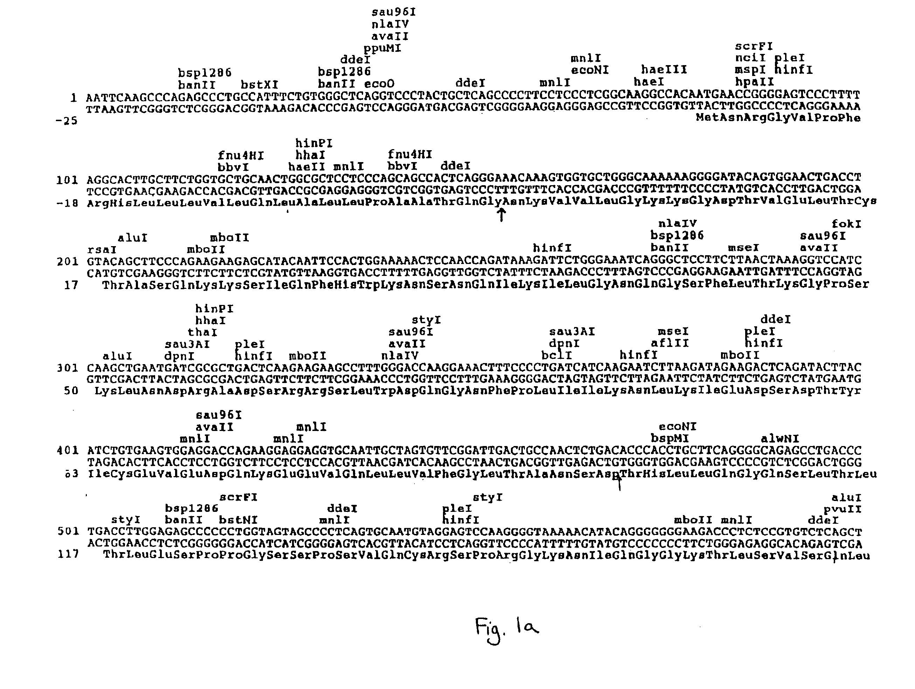

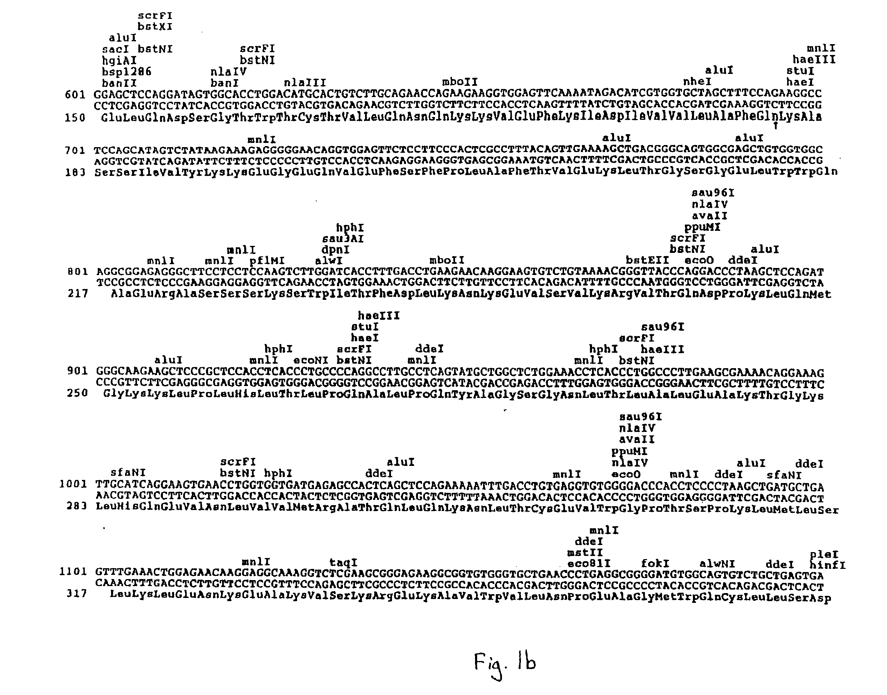

The boundary domains for the CD4 V-like regions (V1-V4) are, respectively, about 100-109, about 175-184, about 289-298, and about 360-369 (based on the precursor CD4 amino acid sequence in which the initiating met is -25; Fig. 1a), CD4 sequences containing any of the CD4 V domains are fused to the immunoglobulin sequence. It is preferable that the V1 domain of CD4 or CD4 lacking the transmembrane and cytoplasmic domains be fused at their C-termini to the immunoglobulin constant region. The precise site at which the fusion is made is not critical; the boundary domains noted herein are for guidance only and other sites neighboring or within the V regions may be selected in order to optimize the secretion or binding characteristics of the CD4. The optimal site will be determined by routine experimentation. In general, it has been found that the fusions are expressed intracellularly, but a great deal of variation is encountered in the degree of secretion of the fusions from recombinant hosts. For instance, the following table demonstrates the various immunoglobulin fusions that have been obtained by the method of this invention. In all examples of CD4 immunoadhesons, the CD4 signal was used to direct secretion from 293 cells. Lower case m represents murine origin, while the lower case h designates human origin. V and C are abbreviations for immunoglobulin variable and constant domains respectively. The numerical subscripts indicate the number of parenthetical units found in the designated multimer. It will be understood that the chains of the multimers are believed to be disulfide bonded in the same fashion as native immunoglobulins. The CD4 immunoadhesons typically contained either the first N-terminal 366 residues of CD4 (CD4₄) or the first 180 N-terminal residues of CD4 (CD4₂) linked at their C-terminus to the κ (light) chain or IgG1 heavy chain constant region (γ1).

**Table I**

| Transfected Gene | Secreted Product |
|---|---|
| mV_{κ}C_{κ} | mV_{κ}C_{κ} and/or (mV_{κ}C_{κ})₂ |
| | |
| mV_{γ1}C_{γ1} | ND |
| | |
| mV_{κ}C_{κ} + µ _{γ1}C_{γ1} | (mV_{κ}C_{κ})₂(mV_{γ1}C_{γ1})₂ + mV_{κ}C_{κ} and/or (mV_{κ} C_{κ})₂ |
| | |
| hCD4-mC_{κ} | hCD4-mC_{κ} and/or (hCD4-mC_{κ} )₂ |
| | |
| hCD4-mC_{γ1} | ND |
| | |
| hCD4-mC_{κ} + hCD4-mC_{γ1} | (hCD4-mC_{κ} )₂(hCD4-mC_{γ1})₂ + hCD4-mC, and/or (hCD4-mC_{κ})₂ |
| | |
| hCD4-hC_{κ} | hCD4-hC_{κ} and/or (hCD4-hC_{κ} )₂ |
| | |
| hCD4-hC_{γ1} | (hCD4-hC_{γ1})₂ |
| | |
| hCD4-hC_{κ} + hCD4-hC_{γ1} | (hCD4-hC_{κ})₂(hCD4-hC_{γ1})₂ + hCD4-hC_{κ} and/or (hCD4-hC_{κ})₂ |
| | |
| mV_{κ} C_{κ} + hCD4-hC_{γ1} | (mV_{κ} C_{κ})₂(hCD4-hC_{γ1})₂ + mV_{κ}C_{κ} and/or (mV_{κ}C_{κ})₂ |
| *ND - Not detected | |

It is interesting to observe from this table that the CD4-human heavy chain immunoadheson was secreted as a dimer whereas the analogous murine construction was not detected (this not excluding the intracellular accumulation of the protein, however). The ability of the hCD4-hCγ1 transformants to produce heavy chain dimer was unexpected since previous work had suggested that immunoglobulin heavy chains are not secreted unless the hosts are cotransformed with nucleic acid encoding both heavy and light chain (Valle et al., Nature 241:338 [1981]). According to this invention, CD4-IgG immunoadheson chimeras are readily secreted wherein the CD4 epitope is present in light chain dimers, heavy chain monomers or dimers, and heavy and light chain tetramers wherein the CD4 epitope is present fused to one or more light or heavy chains, including heterotetramers wherein up to and including all four variable region analogues are derived from CD4. Where light-heavy chain non-CD4 variable domain is present, a heterofunctional antibody thus is provided.

Suitable companion immunoglobulin combining sites and fusion partners are obtained from IgG-1, -2, -3, or 4-subtypes, IgA, IgE, IgD or IgM, but preferably IgG-1.

A preferred embodiment is a fusion of an N-terminal portion of CD4, which contains the binding site for the gp120 envelope protein of HIV, to the C-terminal F_{c} portion of an antibody, containing the effector functions of immunoglobulin G₁. There are two preferred embodiments of this sort; in one, the entire heavy chain constant region is fused to a portion of CD4; in another, a sequence beginning in the hinge region just upstream of the papain cleavage site which defines IgG F_{c} chemically (residue 216, taking the first residue of heavy chain constant region to be 114 [Kobat *et al*., "Sequences of Proteins of Immunological Interest" 4th Ed., 1987], or analogous sites of other immunoglobulins) is fused to a portion of CD4. These embodiments are described in the examples.

More particularly, those variants in which one or more immunoglobulin-like domains of an adheson are substituted for the variable region of an immunoglobulin chain are believed to exhibit improved in vivo plasma half life. These chimeras are constructed in a fashion similar to chimeric antibodies in which a variable domain from a antibody of one species is substituted for the variable domain of another species. See, for example, EP 0 125 023; Munro, Nature 312: (13 December 1984); Neuberger et al., Nature 312: (13 December 1984); Sharon et al., Nature 309: (24 May 1984); Morrison et al., Proc. Natl. Acad. Sci. USA 81:6851-6855 (1984); Morrison et al. Science 229:1202-1207 (1985); and Boulianne et al., Nature 312:643-646 (13 December 1984). The DNA encoding a CD4 V-like region is cleaved by a restriction enzyme at or proximal to a DNA encoding a boundary domain and at a point at or near the DNA encoding N-terminal 5' end of the mature antigen (where use of a non-CD4 leader is contemplated) or at or proximal to the 5' end of the N-terminal coding region for the antigen (where a native CD4 signal is employed). This DNA fragment then is readily inserted into DNA encoding an immunoglobulin light or heavy chain constant region and, if necessary, tailored by deletional mutagenesis. Preferably, this is a human immunoglobulin when the variant is intended for in vivo therapy for humans. DNA encoding immunoglobulin light or heavy chain constant regions is known or readily available from cDNA libraries or is synthesized. See for example, Adams et al., Biochemistry 19:2711-2719 (1980); Gough et al., Biochemistry 19:2702-2710 (1980); Dolby et al., P.N.A.S. USA, 77:6027-6031 (1980); Rice et al., P.N.A.S. USA 79: 7862-7865 (1982); Falkner et al., Nature 298:286-288 (1982); and Morrison et al., Ann. Rev. Immunol. 2:239-256 (1984).

DNA encoding the immunoglobulin or immunoadheson chimeric chain(s) is transfected into a host cell for expression. If the host cell is producing a heavy chain immunoglobulin prior to transfection then one need only transfect with the adheson fused light chain to produce a heteroantibody. Similarly, if the host cell is expressing a light chain then DNA encoding a heavy chain adheson fusion is transfected to produce a heteroantibody. The aforementioned immunoglobulins having one or more arms bearing the adheson domain and one or more arms bearing companion variable regions result in dual specificity for adheson ligand and for an antigen. These are produced by the above-described recombinant methods or by in vitro procedures. In the latter case, for example, F(ab')₂ fragments of the adheson fusion and an immunoglobulin are prepared, the F(ab')2 fragments converted to Fab' fragments by reduction under mild reducing conditions, and then reoxidized in each other's presence under acidic conditions in accord with methods known per se. See also U.S. patent 4,444,878.

In an alternative method for producing a heterofunctional antibody, host cells producing an adheson-immunoglobulin fusion, e.g. transfected myelomas, also are fused with B cells or hybridomas which secrete antibody having the desired companion specificity for an antigen. Heterobifunctional antibody is recovered from the culture medium of such hybridomas, and thus may be produced somewhat more conveniently than by conventional in vitro resorting methods (EP 68,763).

Another group of fusions are those in which an adheson is conjugated with a toxic substance, e.g. a polypeptide such as ricin (including deglycosylated ricin A chain), diptheria toxin A, or a non-peptidyl cytotoxin. Where the toxin is a polypeptide it is convenient to cross-link the polypeptide to the adheson or its transmembrane-deleted variant by conventional in vitro protein cross-linking agents (for suitable methods for linking ricin A chain or deglycosylated A chain to CD4 see, for example, Duncan et al., "Analy. Biochem." 132:68-73 [1983]; Thorpe et al., "Cancer Res." 47: 5924 [1987]; and Ghotre et al., "Cancer Res." 48:2610 [1988]) or by recombinant synthesis as a fusion (see for example, U.S. Patent 4,765,382). Alternatively, where companion antibodies are anti-ricin antibody immunoglobulin variable domains, such immunoglobulin heteroantibodies are employed to deliver ricin to HIV infected cells following the general procedure of Raso et al., Cancer Research, 41:2073 (1981).

Another class of adheson variants are deletional variants. Deletions are characterized by the removal of one or more amino acid residues from a adheson sequence. Typically, the transmembrane and cytoplasmic domains of adhesons ar deleted. In the case of CD4, at least residues 368 to 395 (the transmembrane region), and ordinarily 396-433 as well (the cytoplasmic domain), will be deleted to obtain secreted forms of this adheson. Parenthetically, the amino acid residues follow the numbers given for mature CD4 as noted, for example, in figures 1a - 1c.

Substitutional variants are those in which at least one residue in the adheson sequence has been removed and a different residue inserted in its place. The native N-terminal residue for mature CD4 is now known to be lysine. Thus, the sequence shown in Fig. 1, with an N-terminal asparagine, is an amino acid sequence variant of native mature CD4. Table 2 below describes substitutions which in general will result in fine modulation of the characteristics of the CD antigen.

**TABLE 2**

| Original Residue | Exemplary Substitutions |
|---|---|
| Ala | ser |
| Arg | lys |
| Asn | gln; his |
| Asp | glu |
| Cys | ser; ala |
| Gln | asn |
| Glu | asp |
| Gly | pro |
| His | asn; gln |
| Ile | leu; val |
| Leu | ile; val |
| Lys | arg; gln; glu |
| Met | leu; ile |
| Phe | met; leu; tyr |
| Ser | thr |
| Thr | ser |
| Trp | tyr |
| Tyr | trp; phe |
| Val | ile; leu |

Substantial changes in function or immunological identity are made by selecting substitutions that are less conservative than those in Table 2, i.e., selecting residues that differ more significantly in their effect on maintaining (a) the structure of the polypeptide backbone in the area of the substitution, for example as a sheet or helical conformation, (b) the charge or hydrophobicity of the molecule at the target site or (c) the bulk of the side chain. The substitutions which in general are expected to produce the greatest changes in adheson properties will be those in which (a) a hydrophilic residue, e.g. seryl or threonyl, is substituted for (or by) a hydrophobic residue, e.g. leucyl, isoleucyl, phenylalanyl, valyl or alanyl; (b) a cysteinyl or prolyl is substituted for (or by) any other residue; (c) a residue having an electropositive side chain, e.g., lysyl, arginyl, or histidyl, is substituted for (or by) an electronegative residue, e.g., glutamyl or aspartyl; or (d) a residue having a bulky side chain, e.g., phenylalanyl, is substituted for (or by) one not having a side chain, e.g., glycyl.

A preferred class of substitutional or deletional variants are those involving the transmembrane region of the adheson. The transmembrane region of the adheson is a highly hydrophobic or lipophilic domain that is the proper size to span the lipid bilayer of the cellular membrane. It is believed to anchor the adheson in the cell membrane.

Deletion or substitution of the transmembrane domain will facilitate recovery and provide a soluble form of the adheson by reducing its cellular or membrane lipid affinity and improving its water solubility. If the transmembrane and cytoplasmic domains are deleted one avoids the introduction of potentially immunogenic epitopes, either by exposure of otherwise intracellular polypeptides that might be recognized by the body as foreign or by insertion of heterologous polypeptides that are potentially immunogenic. A principal advantage of the transmembrane deleted adheson is that it is secreted into the culture medium of recombinant hosts. This variant is water soluble and does not have an appreciable affinity for cell membrane lipids, thus considerably simplifying its recovery from recombinant cell culture.

It will be amply apparent from the foregoing discussion that substitutions, deletions, insertions or any combination thereof are introduced to arrive at a final construct. As a general proposition, all variants will not have a functional transmembrane domain and preferably will not have a functional cytoplasmic sequence. This is generally accomplished by deletion of the relevant domain, although adequate insertional or substitutional mutagens also can be effective for this purpose. For example, the transmembrane domain is substituted by any amino acid sequence, e.g. a random or homopolynucleic sequence of about 5 to 50 serine, threonine, lysine, arginine, glutamine, aspartic acid and like hydrophilic residues, which altogether exhibit a hydrophilic hydropathy profile, so that it is secreted into the culture medium of recombinant hosts. This variant should also be considered to be an adheson variant.

These variants ordinarily are prepared by site specific mutagenesis of nucleotides in the DNA encoding the adheson, thereby producing DNA encoding the variant, and thereafter expressing the DNA in recombinant cell culture. However, variant adhesons also are prepared by in vitro synthesis. Obviously, variations made in the DNA encoding the variant adhesons must not place the sequence out of reading frame and preferably will not create complementary regions that could produce secondary mRNA structure deleterious to expression (EP 75,444A). The CD4 variants typically exhibit the same gp120 binding activity as does the naturally-occurring prototype, although variants also are selected in order to modify the characteristics of the CD4 adheson as indicated above.

While the site for introducing an amino acid sequence variation is predetermined, the mutation per se need not be predetermined. For example, in order to optimize the performance of a mutation at a given site, random mutagenesis may be conducted at the target codon or region and the expressed adheson variants screened for the optimal combination of desired activities. Techniques for making substitution mutations at predetermined sites in DNA having a known sequence are well known, for example M13 primer mutagenesis.

Adheson variants that are not capable of binding HIV gp120 are useful nonetheless as immunogens for raising antibodies to the adheson or as immunoassay kit components (labelled, as a competitive reagent for gp120 assay, or unlabelled as a standard for an adheson assay) so long as at least one adheson epitope remains active.

The DNA encoding adhesons is obtained by known procedures. See Reinhertz et al. and Maddon et al., op cit. In general, prokaryotes are used for cloning of CD4 variant DNA sequences. For example, E. coli K12 strain 294 (ATCC No. 31446) is particularly useful. Other microbial strains which may be used include E. coli B, UM101 and E. coli χ1776 (ATCC No. 31537). These examples are illustrative rather than limiting.

DNA encoding the variant adhesons are inserted for expression into vectors containing promoters and control sequences which are derived from species compatible with the intended host cell. The vector ordinarily, but need not, carry a replication site as well as one or more marker sequences which are capable of providing phenotypic selection in transformed cells. For example, E. coli is typically transformed using a derivative of pBR322 which is a plasmid derived from an E. coli species (Bolivar, et al., Gene 2: 95 [1977]). pBR322 contains genes for ampicillin and tetracycline resistance and thus provides easy means for identifying transformed cells. The pBR322 plasmid, or other microbial plasmid must also contain or be modified to contain promoters and other control elements commonly used in recombinant DNA constructions.

Promoters suitable for use with prokaryotic hosts illustratively include the β-lactamase and lactose promoter systems (Chang et al., Nature, 275: 615 [1978]; and Goeddel et al., Nature 281: 544 [1979]), alkaline phosphatase, the tryptophan (trp) promoter system (Goeddel, Nucleic Acids Res. 8: 4057 [1980] and EPO Appln. Publ. No. 36,776) and hybrid promoters such as the tac promoter (H. de Boer et al., Proc. Natl. Acad. Sci. USA 80: 21-25 [1983]). However, other functional bacterial promoters are suitable. Their nucleotide sequences are generally known, thereby enabling a skilled worker operably to ligate them to DNA encoding the adheson variant using linkers or adaptors to supply any required restriction sites (Siebenlist et al., Cell 20: 269 [1980]). Promoters for use in bacterial systems also will contain a Shine-Dalgarno (S.D.) sequence operably linked to the DNA encoding the antigen.

In addition to prokaryotes, eukaryotic microbes such as yeast cultures also are useful as cloning or expression hosts. Saccharomyces cerevisiae, or common baker's yeast is the most commonly used eukaryotic microorganism, although a number of other strains are commonly available. For expression in Saccharomyces, the plasmid YRp7, for example, (Stinchcomb, et al., Nature 282: 39 [1979]; Kingsman et al, Gene 7: 141 [1979]; Tschemper et al., Gene 10: 157 [1980]) is commonly used. This plasmid already contains the trp1 gene which provides a selection marker for a mutant strain of yeast lacking the ability to grow in tryptophan, for example ATCC no. 44076 or PEP4-1 (Jones, Genetics 85: 12 [1977]). The presence of the trp1 lesion as a characteristic of the yeast host cell genome then provides an effective means of selection by growth in the absence of tryptophan.

Suitable promoting sequences for use with yeast hosts include the promoters for 3-phosphoglycerate kinase (Hitzeman et al., J. Biol. Chem. 255: 2073 [1980]) or other glycolytic enzymes (Hess et al., J. Adv. Enzyme Reg. 7: 149 [1968]; and Holland, Biochemistry 17: 4900 [1978]), such as enolase, glyceraldehyde-3-phosphate dehydrogenase, hexokinase, pyruvate decarboxylase, phosphofructokinase, glucose-6-phosphate isomerase, 3-phosphoglycerate mu-tase, pyruvate kinase, triosephosphate isomerase, phosphoglucose isomerase, and glucokinase.

Other yeast promoters, which are inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein, glyceraldehyde-3-phosphate dehydrogenase, and enzymes responsible for maltose and galactose utilization. Suitable vectors and promoters for use in yeast expression are further described in R. Hitzeman et al., European Patent Publication No. 73,657A. Yeast enhancers also are advantageously used with yeast promoters.

Promoters are controlling transcription from vectors in mammalian host cells may be obtained from various sources, for example, the genomes of viruses such as: polyoma, Simian Virus 40 (SV40), adenovirus, retroviruses, hepatitis-B virus and most preferably cytomegalovirus, or from heterologous mammalian promoters, e.g. the beta actin promoter. The early and late promoters of the SV40 virus are conveniently obtained as an SV40 restriction fragment which also contains the SV40 viral origin of replication. Fiers et al., Nature, 273: 113 (1978). The immediate early promoter of the human cytomegalovirus is conveniently obtained as a Hindlll E restriction fragment. Greenaway, P.J. et al., Gene 18: 355-360 (1982). Of course, promoters from the host cell or related species also are useful herein.

DNA transcription in higher eukaryotes is increased by inserting an enhancer sequence into the vector. Enhancers are cis-acting elements of DNA, usually from about 10 to 300bp, that act to increase the transcription initiation capability of a promoter. Enhancers are relatively orientation and position independent having been found 5' (Laimins, L. et al., Proc.Natl.Acad.Sci. 78: 993 [1981]) and 3' (Lusky, M.L., et al., Mol. Cell Bio. 3: 1108 [1983]) to the transcription unit, within an intron (Banerji, J.L. et al., Cell 33: 729 [1983]) as well as within the coding sequence itself (Osborne, TF., et al., Mol. Cell Bio. 4: 1293 [1984]). Many enhancer sequences are now known from mammalian genes (globin, elastase, albumin, α-fetoprotein and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers.

Expression vectors used in eukaryotic host cells (yeast, fungi, insect, plant, animal, human or nucleated cells) may also contain sequences necessary for the termination of transcription which may affect mRNA expression. These regions are transcribed as polyadenylated segments in the untranslated portion of the mRNA encoding the adheson.

Expression vector systems generally will contain a selection gene, also termed a selectable marker. Examples of suitable selectable markers for mammalian cells are dihydrofolate reductase (DHFR), thymidine kinase or neomycin. When such selectable markers are successfully transferred into a mammalian host cell, the transformed mammalian host cell can survive if placed under selective pressure. There are two widely used distinct categories of selective regimes. The first category is based on a cell's metabolism and the use of a mutant cell line which lacks the ability to grow independent of a supplemented medium. Two examples are: CHO DHFR⁻ cells and mouse LTK⁻ cells. These cells lack the ability to grow without the addition of such nutrients as thymidine or hypoxanthine. Because these cells lack certain genes necessary for a complete nucleotide synthesis pathway, they cannot survive unless the missing nucleotides are provided in a supplemented medium. An alternative to supplementing the medium is to introduce an intact DHFR or TK gene into cells lacking the respective genes, thus altering their growth requirements. Individual cells which were not transformed with the DHFR or TK gene will not be capable of survival in non supplemented media.

The second category is dominant selection which refers to a selection scheme used in any cell type and does not require the use of a mutant cell line. These schemes typically use a drug to arrest growth of a host cell. Those cells which have a novel gene would express a protein conveying drug resistance and would survive the selection. Examples of such dominant selection use the drugs neomycin, Southern P. and Berg, P., J. Molec. Appl. Genet. 1: 327 (1982), mycophenolic acid, Mulligan, R.C. and Berg, P. Science 209: 1422 (1980) or hygromycin, Sugden, B. et al., Mol. Cell. Biol. 5: 410-413 (1985). The three examples given above employ bacterial genes under eukaryotic control to convey resistance to the appropriate drug G418 or neomycin (geneticin), xgpt (mycophenolic acid) or hygromycin, respectively.

"Amplification" refers to the increase or replication of an isolated region within a cell's chromosomal DNA. Amplification is achieved using a selection agent e.g. methotrexate (MTX) which inactivates DHFR. Amplification or the making of successive copies of the DHFR gene results in greater amounts of DHFR being produced in the face of greater amounts of MTX. Amplification pressure is applied notwithstanding the presence of endogenous DHFR, by adding ever greater amounts of MTX to the media. Amplification of a desired gene can be achieved by cotransfecting a mammalian host cell with a plasmid having a DNA encoding a desired protein and the DHFR or amplification gene permitting cointegration. One ensures that the cell requires more DHFR, which requirement is met by replication of the selection gene, by selecting only for cells that can grow in the presence of ever-greater MTX concentration. So long as the gene encoding a desired heterologous protein has cointegrated with the selection gene replication of this gene gives rise to replication of the gene encoding the desired protein. The result is that increased copies of the gene, i.e. an amplified gene, encoding the desired heterologous protein express more of the desired heterologous protein.

Preferred host cells for expressing the CD antigen variants of this invention are mammalian cell lines, examples including: monkey kidney CV1 line transformed by SV40 (COS-7, ATCC CRL 1651); human embryonic kidney line (293, Graham, F.L. et al. J. Gen Virol. 36: 59 [1977] and 293s cells [293 subclones selected for better suspension growth]); baby hamster kidney cells (BHK, ATCC CCL 10): chinese hamster ovary-cells-DHFR (CHO, Urlaub and Chasin, Proc.Natl.Acad.Sci. (USA) 77: 4216, [1980]); mouse sertoli cells (TM4, Mather, J.P., Biol. Reprod. 23: 243-251 [1980]); monkey kidney cells (CV1 ATCC CCL 70): african green monkey kidney cells (VERO-76, ATCC CRL-1587); human cervical carcinoma cells (HELA, ATCC CCL 2): canine kidney cells (MDCK, ATCC CCL 34): buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51 cells); and TRI cells (Mather, J.P. et al., Annals N.Y. Acad. Sci. 383: 44-68 [1982]).

"Transformation" means introducing DNA into an organism so that the DNA is replicable, either as an extrachromosomal element or by chromosomal integration. One suitable for transformation of the host cells is the method of Graham, F. and van der Eb, A., Virology 52: 456-457 (1973). However, other methods for introducing DNA into cells such as by nuclear injection or by protoplast fusion may also be used. If prokaryotic cells or cells which contain substantial cell walls are used as hosts, the preferred method of transfection is calcium treatment using calcium chloride as described by Cohen, F.N. et al., Proc. Natl. Acad. Sci. (USA), 69: 2110 (1972).

Construction of suitable vectors containing the desired coding and control sequences employ standard and manipulative ligation techniques. Isolated plasmids or DNA fragments are cleaved, tailored, and religated in the form desired to form the plasmids required. Suitable procedures are well known for the construction described herein. See, for example, (Man iatis, T. et al., Molecular Cloning, 133-134 Cold Spring Harbor, [1982]; "Current Protocols in Molecular Biology", edited by Ausubel *et al*., [1987], pub. by Greene Publishing Associates & Wiley-Interscience).

Correct plasmid sequences are confirmed by transforming E. coli K12 strain 294 (ATCC 31446) with ligation mixtures, successful transformants selected by ampicillin or tetracycline resistance where appropriate, plasmids from the transformants prepared, and then analyzed by restriction enzyme digestion and/or sequenced by the method of Messing et al., Nucleic Acids Res. 9: 309 (1981) or by the method of Maxam et al., Methods in Enzymology 65: 499 (1980).

Host cells are transformed with the expression vectors of this invention. Thereafter they are cultured in appropriate culture media, e.g. containing substances for inducing promoters, selecting transformants or amplifying genes. The culture conditions, such as temperature, pH and the like, are those previously used with the host cell selected for expression, and will be apparent to the ordinarily skilled artisan.

The secreted adheson variants are recovered and purified from the culture supernatants of recombinant hosts. Typically, the supernatants are concentrated by ultrafiltration, contacted with a ligand affinity or immunoaffinity matrix so as to adsorb the adheson variant, and eluted from the matrix. Optionally, the adheson is purified by ion exchange chromatography.

Surprisingly, purification of soluble CD4 adheson from culture medium was unexpectedly difficult. Notwithstanding that the hydrophobic transmembrane region of the antigen had been deleted, the antigen exhibited a strong tendency to form aggregates that could be readily removed from suspension by centrifugation at 1000 x g, and which avidly coat surfaces such as ultrafiltration membranes. This appears to result from the reduction in concentration of albumin or other serum protein (ordinarily present in the crude preparation) to a particular level, below which the truncated antigen no longer remains soluble. This phenomenon appears to be aggravated by exposure of the CD4 adheson to low pH (< about pH 4). As a result, separation procedures (particularly those that employ acid elution, such as immunoaffinity) should be modified so that the eluate is maintained at, or immediately returned to, about neutrality. Further, a surfactant, e.g. a detergent such as Tween 80, should be included with the antigen during the separation procedure. The final purified product will be stabilized with a predetermined protein such as albumin, and/or a detergent.

The purified adheson is formulated into conventional pharmacologically acceptable excipients.

It is administered to patients having HIV infection at a dosage capable of maintaining a concentration of greater than about 100 ng of soluble CD4 adheson/ml plasma. For CD4 adheson variants having different molecular weights, about 2 picomoles of soluble receptor per ml of plasma will be initially evaluated clinically in order to establish a stoichiometric equivalence with native (membrane bound) and soluble receptor. The ordinary dosage of soluble CD4 is 100 µg/kg of patient weight/day.

The therapeutic CD4 variants are employed with other therapies and agents for the treatment of AIDS, including AZT, neutralizing antibodies and immunocytotoxins, gp120 fragments and vaccines.

In order to facilitate understanding of the following examples certain frequently occurring methods and/or terms will be described.

"Plasmids" are designated by a lower case p preceded and/or followed by capital letters and/or numbers. The starting plasmids herein are either commercially available, publicly available on an unrestricted basis, or can be constructed from available plasmids in accord with published procedures. In addition, equivalent plasmids to those described are known in the art and will be apparent to the ordinarily skilled artisan.

"Digestion" of DNA refers to catalytic cleavage of the DNA with a restriction enzyme that acts only at certain sequences in the DNA. The various restriction enzymes used herein are commercially available and their reaction conditions, cofactors and other requirements were used as would be known to the ordinarily skilled artisan. For analytical purposes, typically 1 µg of plasmid or DNA fragment is used with about 2 units of enzyme in about 20 µl of buffer solution. For the purpose of isolating DNA fragments for plasmid construction, typically 5 to 50 µg of DNA are digested with 20 to 250 units of enzyme in a larger volume. Appropriate buffers and substrate amounts for particular restriction enzymes are specified by the manufacturer. Incubation times of about 1 hour at 37°C are ordinarily used, but may vary in accordance with the supplier's instructions. After digestion the reaction is electrophoresed directly on a polyacrylamide gel to isolate the desired fragment.

"Recovery" or "isolation" of a given fragment of DNA from a restriction digest means separation of the digest on polyacrylamide or agarose gel by electrophoresis, identification of the fragment of interest by comparison of its mobility versus that of marker DNA fragments of known molecular weight, removal of the gel section containing the desired fragment, and separation of the gel from DNA. This procedure is known generally (Lawn, R. et al., Nucleic Acids Res. 9: 6103-6114 [1981], and Goeddel, D. et al., Nucleic Acids Res. 8: 4057 [1980]).

"Dephosphorylation" refers to the removal of the terminal 5' phosphates by treatment with bacterial alkaline phosphatase (BAP). This procedure prevents the two restriction cleaved ends of a DNA fragment from "circularizing" or forming a closed loop that would impede insertion of another DNA fragment at the restriction site. Procedures and reagents for dephosphorylation and other recombinant manipulations are conventional. Reactions using BAP are carried out in 50 mM Tris at 68°C to suppress the activity of any exonucleases which may be present in the enzyme preparations. Reactions were run for 1 hour. Following the reaction the DNA fragment is gel purified.

"Ligation" refers to the process of forming phosphodiester bonds between two double stranded nucleic acid fragments (Maniatis, T. et al., Id. at 146). Unless otherwise provided, ligation may be accomplished using known buffers and conditions with 10 units of T4 DNA ligase ("ligase") per 0.5 µg of approximately equimolar amounts of the DNA fragments to be ligated.

"Filling" or "blunting" refers to the procedures by which the single stranded end in the cohesive terminus of a restriction enzyme-cleaved nucleic acid is converted to a double strand. This eliminates the cohesive terminus and forms a blunt end. This process is a versatile tool for converting a restriction cut end that may be cohesive with the ends created by only one or a few other restriction enzymes into a terminus compatible with any blunt-cutting restriction endonuclease or other filled cohesive terminus. Typically, blunting is accomplished by incubating 2-15µg of the target DNA in 10mM MgCl₂, 1mM dithiothreitol, 50mM NaCl, 10mM Tris (pH 7.5) buffer at about 37°C in the presence of 8 units of the Klenow fragment of DNA polymerase I and 250 µM of each of the four deoxynucleoside triphosphates. The incubation generally is terminated after 30 min. phenol and chloroform extraction and ethanol precipitation.

The following examples merely illustrate the best mode now contemplated for practicing the invention, but should not be construed to limit the invention. All literature citations herein are expressly incorporated by reference.

### Example 1

### Construction of Vectors for the Expression of Native CD4 and Secreted Derivatives

### Section 1

The plasmid used for recombinant synthesis of human CD4 was pSVeCD4DHFR. The plasmid was constructed as follows:

λCD4P1 containing most of the coding sequence of human CD4 (obtained from a human placental cDNA library using oligonucleotide probes based on the published sequence [Maddon et al. 1985]) was digested with EcoRI to produce the cDNA insert. This fragment was recovered by polyacrylamide gel electrophoresis (fragment 1).

pUC18 was digested with EcoRl and the single fragment recovered by polyacrylamide gel electrophoresis (fragment 2). Fragment 1 was ligated to fragment 2 and the ligation mixture transformed into E. coli strain 294. The transformed culture was plated on ampicillin media plates and resistant colonies selected. Plasmid DNA was prepared from transformants and checked by restriction analysis for the presence of the correct DNA fragments. This plasmid is referred to as pUCCD4.

pSVeE'DHFR (Muesing et al., Cell 48:691-701 [1987]) was digested with Kpnl and BamHl and blunted with E. coli DNA polymerase I (Klenow fragment) and the four dNTPs. Fragment 3 containing the pML-Amp^{r} region, SV40 early promoter, the HIV LTR, and the mouse DHFR gene was recovered by gel electrophoresis, ligated and the ligation mixture transformed into E. coli strain 294. The transformed culture was plated on ampicillin media plates and resistant colonies selected. Plasmid DNA was prepared from transformants and checked by restriction analysis for the presence of the BamHl restriction site and the absence of the Kpnl restriction site. This plasmid is referred to as pSVeΔBKDHFR and allows EcoRl-BamHl fragments to be inserted after the SV40 early promoter and transcribed under its control, following transfection into an appropriate cell line.

Synthetic oligonucleotides (adaptors 1-8, below) were made to extend from 76 bp 5' of the initiation codon of CD4 translation to the Rsal restriction site at 121 bp 3' of the initiator, with the sequence AATT at the 5' end of the sense strand to generate an end which could ligate to an EcoRl restriction fragment. These oligonucleotides were ligated and the 204 bp fragment containing the entire sequence recovered by gel electrophoresis (fragment 4).

CD4 adaptor 1: AATTCAAGCCCAGAGCCCTGCCATTTCTGTGGGCTCAGGTCCCT

CD4 adaptor 2: pACTGCTCAGCCCCTTCCTCCCTCGGCAAGGCCACAATGAACCGGGGAGTC

CD4 adaptor 3: pCCTTTTAGGCACTTGCTTCTGGTGCTGCAACTGGCGCTCCTCCCAGC

CD4 adaptor 4:
pAGCCACTCAGGGAAACAAAGTGGTGCTGGGCAAAAAAGGGGATACAGTGGAACTGACCTGT

CD4 adaptor 5: pACAGGTCAGTTCCACTGTATCCCCTTTTTTGCCCAGCACCACTTTGTTTCC

CD4 adaptor 6: pCTGAGTGGCTGCTGGGAGGAGCGCCAGTTGCAGCACCAGAAGCAAGT

CD4 adaptor 7: pGCCTAAAAGGACTCCCCGGTTCATTGTGGCCTTGCCGAGGGAGGAAGGG

CD4 adaptor 8:  GCTGAGCAGTAGGGACCTGAGCCCACAGAAATGGCAGGGCTCTGGGCTTG

pUCCD4 was digested with Rsal and Sstl and the 401 bp fragment containing part of the CD4 coding sequence recovered by gel electrophoresis (fragment 5). pUC18 was digested with EcoRI and Sstl and the fragment comprising the bulk of the plasmid recovered by gel electrophoresis (fragment 6). Fragments 4 and 5 were ligated to fragment 6 and the ligation mixture transformed into E. coli strain 294. The transformed culture was plated on ampicillin media plates and resistant colonies selected. Plasmid DNA was prepared from transformants and checked by restriction analysis for the presence of the correct fragment. The sequence of the inserted synthetic DNA was checked by excising the 605 bp EcoRI-Sstl fragments from several transformants and ligating them to M13mp19 which had been digested with the same enzymes. After transformation into E. coli strain JM101, single-stranded DNA was prepared and sequenced. One plasmid which contained the correct sequence was selected, and is referred to as pCD4int.

pCD4int was digested with EcoRl and Sstl and fragment 7 containing the 5' end of the CD4 coding region was recovered by gel electrophoresis. pUCCD4 was digested with Sstl and BamHl and the 1139 bp fragment containing the remainder of the CD4 coding region (fragment 8) recovered by gel electrophoresis.

pSVeΔBKDHFR was digested with EcoRI and BamHl and fragment 9 comprising the bulk of the plasmid was isolated. Fragments 7, 8 and 9 were ligated and the ligation mixture transformed into E. coli strain 294. The transformed culture was plated on ampicillin media plates and the resistant colonies selected. Plasmid DNA was prepared from transformants and checked by restriction analysis for the presence of the correct fragment. This plasmid is referred to as pSVeCD4DHFR, and was used to direct synthesis of recombinant intact CD4.

### Section 2

A plasmid was constructed to direct the synthesis of a CD4 derivative lacking the putative transmembrane domain and most of the putative cytoplasmic domain (Maddon et al.). This was done with the intention of creating a secreted form of CD4, based on the assumption that these domains anchor the CD4 glycoprotein to the cell membrane, and that their deletion would result in the secretion of the product. This plasmid is referred to as pSVeCD4ΔN1aDHFR and was constructed as follows:

pUCCD4 was digested with Sstl and Taql and the 531 bp fragment (fragment 10) recovered. pUCCD4 was digested with Nlalll and Taql and the 112 bp fragment (fragment 11) recovered. pUCCD4 was digested with BamHl and Nlalll and the 301 bp fragment (fragment 12) recovered. pCD4int was digested with Sstl and BamHl and fragment 13 comprising the bulk of the plasmid recovered. Fragments 10,11, and 12 were ligated together with fragment 13 and the ligation mixture transformed into E. coli strain 294. The transformed culture was plated on ampicillin media plates and resistant colonies selected. Plasmid DNA was prepared from transformants and checked by restriction analysis for the presence of the correct fragment. Plasmid DNA from several transformants was sequenced to ensure that the 195 bp Nlalll fragment had been deleted and that the proper reading frame was restored. The resulting plasmid is referred to as pCD4ΔN1a.

pCD4ΔN1a was digested with EcoRI and BamHl and the 1541 bp fragment containing the sequence of a CD4 derivative lacking the transmembrane and cytoplasmic domains recovered (fragment 14) and ligated to fragment 9 and the ligation mixture transformed into E. coli strain 294. The transformed culture was plated on ampicillin media plates and resistant colonies selected. Plasmid DNA was prepared from transformants and checked by restriction analysis for the presence of the correct fragment. This plasmid is referred to as pSVeCD4ΔN1aDHFR.

Both pSVeCD4DHFR and pSVeCD4ΔN1aDHFR were transfected into CHO cells by the same method used to establish cell lines stably expressing HIV-I polypeptides (Muesing, Smith and Capon, Cell 48:6910701 [1987]). These cells were assayed for production by radioimmunoprecipitation as described below. While no product was detected in initial experiments, subsequent experiments showed that the above described coding segment could indeed direct the synthesis of a soluble CD4 adheson variant both in CHO and 293 cells.

### Section 3

A different expression system was initially used for the synthesis and expression of a CD4 variant lacking completely the cytoplasmic and transmembrane domains. The system uses the cytomegalovirus promoter and can be used in cultured cells of human origin. The first plasmid constructed for use in this system contained the entire coding region for CD4 and was intended to function as a control in the following studies. It is referred to as pRKCD4, and was constructed as follows:

pSVeCD4DHFR was digested with EcoRI and BamHl and fragment 15 containing the entire CD4 coding region was isolated. pRK5 (European Application No. 88308386.7) was digested with EcoRI and BamHl and fragment 16 comprising the bulk of the plasmid recovered by gel electrophoresis, ligated to fragment 15, and the ligation mixture transformed into E. coli strain 294. The transformed culture was plated on ampicillin media plates and resistant colonies selected. Plasmid DNA was prepared from transformants and checked by restriction analysis for the presence of the correct fragment. This plasmid is referred to as pRKCD4.

### Section 4

The next plasmid constructed was designed to direct the expression of the above-mentioned (Section 3) secreted derivative of CD4. The coding region of CD4 was fused after amino acid residue 368 of mature CD4 to a sequence from pBR322 which codes for 9 more residues before a translation termination codon. This removes the putative CD4 transmembrane and cytoplasmic domains, which are presumed to anchor CD4 to the cell surface. The plasmid is referred to as pRKCD4T, and was constructed as follows:

pSVeCD4DHFR was digested with Hpall, blunted with Klenow fragment and the four dNTPs, and digested with BstEll. The 382 bp fragment (fragment 17) containing part of the CD4 coding sequence was recovered by gel electrophoresis. pSVeCD4DHFR was digested with EcoRl and BstEll and the 874 bp fragment (fragment 18) recovered. pBR322 was digested with Hindlll, blunted with Klenow fragment and the four dNTPs, and digested with EcoRl. Fragment 19 comprising the bulk of the plasmid was isolated and ligated to fragments 17 and 18 and the ligation mixture transformed into E. coli strain 294. The transformed culture was plated on ampicillin media plates and resistant colonies selected. Plasmid DNA was prepared from transformants and checked by restriction analysis for the presence of the correct fragment. This plasmid is referred to as pCD4Tint.

pRK5 was digested with EcoRl and Smal and fragment 20 comprising the bulk of the plasmid isolated. pCD4Tint was digested with EcoRl and EcoRV and the 1410 bp fragment containing the CD4 coding sequence to the Hpall site at 1176 bp 3' of the initiating codon and the 154 bp Hindlll-EcoRV fragment of pBR322 was recovered (fragment 21). Fragments 20 and 21 were ligated and the ligation mixture transformed into E. coli strain 294. The transformed culture was plated on ampicillin media plates and resistant colonies selected. Plasmid DNA was prepared from transformants and checked by restriction analysis for the presence of the correct fragment. This plasmid is referred to as pRKCD4T.

### Section 5a

In order to create a secreted form of CD4 which could be purified with an antibody directed to herpes virus type I glycoprotein D, a plasmid was constructed to express a derivative of CD4T in which the region coding for the mature, processed CD4T polypeptide was fused to a sequence coding for the signal peptide and the first 27 residues of the mature type I Herpes Simplex Virus gD glycoprotein. This plasmid is referred to as pRKGDCD4T, and was constructed as follows:

pgDTrunc.DHFR was digested with EcoRI and Pvull and the fragment containing the coding region for the signal peptide and first 27 residues of the mature HSV I gD glycoprotein was isolated (fragment 22). pRKCD4T was digested with EcoRI and BstEll and fragment 23 containing the 3' end of the CD4 coding sequence and the pRK5 region was isolated.

Synthetic oligonucleotides GD (adaptors 1-2, below) containing the coding sequence of CD4 from the codon for the amino terminal residue of mature CD4 to the Rsa site at 121 bp 3' of translation initiation, and containing the sequence CTGCTCGAG at the 5' end of the sense strand were prepared (fragment 24). pRKCD4 was digested with Rsal and BstEII and the 665 bp fragment containing part of the coding region for CD4 was recovered (fragment 25) and ligated to fragment 24. After digestion with BstEll to ensure that only monomeric fragment was present, the 724 bp fragment containing both sequences was recovered by gel electrophoresis (fragment 26).

Fragments 22, 23 and 26 were ligated and the ligation mixture transformed into E. coli strain 294. The transformed culture was plated on ampicillin media plates and resistant colonies selected. Plasmid DNA was prepared from transformants and checked by restriction analysis for the presence of the correct fragment. The sequence of several transformants was checked to ensure that the synthetic insert was correct and that reading frame was preserved. This plasmid is referred to as pRKGDCD4T.

These pRK5 derived plasmids preferably were transfected into 293S cells for stable expression according to Muesing, et al. Cell 48:691 (1987) with the exception that in addition to the plasmid of interest a plasmid expressing the neomycin resistance gene pRSV neo (Gorman et al. Science 221:553-555 (1985)) was cotransfected. 293 cells also are used satisfactorily as host cells. 2 days after transfection, the cells were passaged into standard medium (1:1 F12/DME supplemented with L-glutamine, penicillin-streptomycin and 10% FBS) with 0.5 mg/ml G418 (Genticin sulfate; Gibco) for selection of stable cell lines, rather than in media containing methotrexate as shown by Muesing et al. Cells were assayed for production of CD4 or CD4 analogs by radioimmunoprecipitation. Binding studies (section 5c) used conditioned supernatants from these cells in the 1:1 F12/DME medium. Materials used in infectivity assays (section 5b) were obtained as described in section 8 below.

gDCD4 adaptor 1:
CTGCTCGAGCAGGGAAACAAAGTGGTGCTGGGCAAAAAAGGGGATACAGTGGAACTGAC

gDCD4 adaptor 2:
pACAGGTCAGTTCCACTGTATCCCCTTTTTTGCCCAGCACCACTTTGTTTCCCTGCTCGA

### Section 5b

The following constitutes a study of the neutralization of HIV-1 infectivity by soluble CD4 analogs. A modification of the neutralization procedure of Robert-Guroff et al., Nature 316:72 (1985) was followed. Equal volumes of inhibitor supernatant and virus (60 microliters) were incubated at 4 degrees C for 1 hour, then the same volume of H9 (Gallo et al., Science 224:500, 1984) at 5x10⁶/ml was added and incubation continued for 1 hour at 37 degrees C. Following absorption, 2.5x10⁵ cells in 150 microliters were transferred to 2 ml of incubation media. After 4 days at 37 degrees C, the cultures were split 1:2 with fresh media and incubated for an additional 3 days. Cultures were harvested, reverse transcriptase activity was measured (Groopman et al., AIDS Research and Human Retroviruses 3:71, 1987), and immunofluorescence reactivity with HIV-1 positive serum was determined as described (Poiesz et al., Proc. Acad. Nat. Sci. USA 77:7415, 1980). Inhibitor supernatants were obtained from confluent plate cultures of 293s/CDT4, 293s/gDCD4T cells or untransfected 293s cells by replacing the growth medium incubation media and harvesting the supernatants 24 hours later. Inhibitor supernatant replaced part or all of the incubation media during the first three days of culture as indicated in the second column of Table 3. Challenge dose of virus was 100 TCID₅₀ (Groopman et al., supra) of HIV-1 strain HTLV-IIIB grown in H9 cells assayed in the same system. Incubation media consisted of RPMI 1640 media containing 2mM L-glutamine, 100 units/ml penicillin, 100 micrograms/ml streptomycin, 2 micrograms/ml polybrene and 20% fetal calf serum (M.A. Bioproducts).

**Table 3**

| Inhibitor supematant | Dilution of Inhibitor supernatant | Indirect **immunofluorescence** (% positive cells) | | Reverse **transcriptase** (cpm/ml x 10⁵) | |
|---|---|---|---|---|---|
| mock-transfected | undil.; 1:4 | 65.3 | 65.5 | 21.8 | 23.9 |
| mock-transfected | undil.; 1:4 | 61.2 | 61.1 | 18.5 | 28.1 |
| CD4T | undil.; 1:4 | 0.4 | 18.0 | 0.11 | 5.94 |
| CD4T | undil.; 1:4 | 0.8 | 16.1 | 0.15 | 3.72 |
| gDCD4T | undil.; 1:4 | 0.4 | 26.8 | 0.14 | 9.92 |
| gDCD4T | undil.; 1:4 | 1.4 | 36.1 | 0.23 | 11.3 |

Both forms of soluble CD4 virtually abolished the growth of HIV-1, when incubated with virus-infected cells without prior dilution (Table 2). At a dilution of 1:4 the soluble CD4 preparations were only partially effective in inhibiting virus growth, however the level of fluorescent-positive cells and reverse transcriptase was still significantly lower than cultures receiving mock-transfected cell supernatants (Table 2). Since there was no significant difference in virus growth between diluted and undiluted control supernatants, nor did any of the supernatants affect the growth of uninfected H9 cells (data not shown), soluble CD4 proteins present in these supernatants were concluded to be responsible for the neutralization of HIV-1 infection of H9 cells.

### Section 5c

To determine the affinity constant for interactions between gp120 and CD4 or CD4 variants, saturation binding analysis was carried out with soluble CD4 (supra) and detergent solubilized intact CD4 (Lasky et al. Cell 50:975 [1987]) employing radioiodinated gp120 labeled with lactoperoxidase. Binding reactions consisted of ¹²⁵I-gp120 (3 ng to 670 ng, 2.9 nCi/ng) incubated for 1 hour at 0 degrees C with cell lysates containing intact CD4 (Laskey et al., op cit.) or cell supernatants containing unlabeled CD4T or gDCD4T prepared as described in section 5a. Reactions (0.2ml) had a final composition of 0.5X McDougal Lysis Buffer (McDLB) (1 x McDLB contains0.5 % Nonidet NP-40, 0.2% Na deoxycholate, 0.12 M NaCl, 0.02 M Tris-HCl, pH 8.0) and were performed in duplicate, both in the presence or absence of 50 micrograms of unlabeled purified gp120 (74 fold or greater excess). Following incubation, bound gp120 was quantitated by immunoprecipitation and counted in a gamma counter. For immunoprecipitation, binding reaction solutions were preabsorbed with 5 microliters of normal rabbit serum for one hour at 0°C, and cleared with 40 microliters of Pansorbin (10 % w/v, Calbiochem) for 30 minutes at 0 degrees C. Samples were then incubated overnight at 0 degrees C with 2 microliters of normal serum or 5 microliters (0.25 microgram) of OKT4 monoclonal antibody (Ortho) followed by collection of immune complexes with 10 microliters of Pansorbin. Precipitates were washed twice in 1X McDLB and once in water, then eluted by eluting at 100 degrees C for 2 minutes in sample buffer (0.12 M Tris-HCl pH 6.8, 4% SDS, 0.7 M mercaptoethanol, 20% glycerol, and 0.1% bromophenol blue). CD4 molecules were bound saturably by gp120, and yielded a simple mass action binding curve. Supernatants from mock-transfected cells gave a level of specifically bound gp120 less than 1% that found for supernatants containing soluble CD4. Scatchard analysis revealed a single class of binding sites on each molecule, with apparent dissociation constants (Kd) of 1.3 x 10⁻⁹ M, 0.83 x 10⁻⁹ M and 0.72 x 10⁻⁹ M for intact CD4, CD4T and gDCD4T, respectively. The values obtained for CD4-gp120 binding in solution are comparable to the affinity previously measured for gp120 binding to CD4 on whole cells (Kd=4.0 x 10⁻⁹ M. Lasky, Cell, supra).

### Section 6

In order to produce secreted derivatives of CD4 which are free of extraneous amino acid residues, two plasmids were constructed for expression in 293 cells. The plasmids contain CD4 genes which have been truncated without the addition of extra residues, and are referred to as pRKCD4ΔN1a and pRKCD4TP, and were constructed as follows:

Fragment 14 containing the CD4 gene with the 195 bp N1alll restriction fragment deleted was ligated to fragment 16, which is pRK5 digested with EcoRI and BamHl. The ligation mixture was transformed into E. coli strain 294, the transformed culture plated on ampicillin media plates and resistant colonies selected. Plasmid DNA was prepared from transformants and checked by restriction analysis for the presence of the correct fragment. The resulting plasmid is referred to as pRKCD4ΔN1a.

Synthetic DNA was made to attach to the Hpall site at 1176bp and which when so attached would terminate translation after amino acid residue 370 of mature CD4 (fragment 27). The other end of this fragment was designed to ligate to BamHl restriction fragments. pUCCD4 was digested with BstEII and Hpall and the 382bp fragment containing part of the CD4 gene was recovered (fragment 28). Fragments 27 and 28 were ligated and then digested with BstEll to reduce dimerized fragments to monomers, and the resulting 401bp fragment was recovered (fragment 29).

pRKCD4 was digested with Bstll and BamHl and the fragment comprising the bulk of the plasmid (fragment 30) was isolated and ligated to fragment 29. The ligation mixture was transformed into E. coli strain 294, the transformed culture plated on ampicillin media plates and resistant colonies selected. Plasmid DNA was prepared from transformants and checked by restriction analysis for the presence of the correct fragment. The resulting plasmid is referred to as pRKCD4TP. Both plasmids are transfected into 293 cells to generate stable variant CD4-expressing cell lines as described above.

### Section 7

Two plasmids were constructed to direct the expression of secreted CD4 lacking extraneous amino acid residues in CHO cells. These are referred to as pSVeCD4ΔN1aSVDHFR and pSVeCD4TPSVDHFR, and were constructed as follows:

pE34BHBV.E400D22 was digested with Pvul and EcoRI and the fragment containing the SV40 early promoter and part of the β-lactamase gene was recovered (fragment 31). pE348HBV.E400D22 was digested with Pvul and BamHl and the large fragment containing the balance of the β-lactamase gene as well as the SV40 early promoter and the DHFR gene was isolated (fragment 32).

Fragments 31 and 32 were ligated together with fragment 14 and transformed into E. coli strain 294. The transformed culture was plated on ampicillin media plates and resistant colonies selected. Plasmid DNA was prepared from transformants and checked by restriction analysis for the presence of the correct fragment. The resulting plasmid is referred to as pSVECD4ΔN1aSVDHFR. This plasmid contains the same DNA fragment encoding the soluble CD4 molecule found in the above-mentioned plasmid pSVeCD4ΔN1aDHFR (Section 2).

pRKCD4TP was digested with EcoRl and BamHl and the fragment containing the truncated CD4 coding region was isolated and ligated to fragments 31 and 32. The ligation mixture was transformed into E. coli strain 294, the transformed culture plated on ampicillin media plates and resistant colonies selected. Plasmid DNA was prepared from transformants and checked by restriction analysis for the presence of the correct fragment. The resulting plasmid is referred to as pSVeCD4TPSVDHFR. Both of these plasmids are transfected into CHO cells and amplified transfectants selected by methotrexate using conventional procedures.

### Example 2

Fusions of the V region of the CD4 gene, which is homologous to the variable region of immunoglobulin genes (ref Maddon et al. 1985), to the constant (C) region of human immunoglobulin κ and γ2 chains are constructed as follows:

Synthetic DNA is made to code for the C region of human κ chain (residues 109-214) based on the sequence published by Morin et al., Proc. Natl. Acad. Sci. 82:7025-7029, with the addition at the 5' end of the coding strand of the sequence GGGG, which allows this fragment to be ligated to the BspMl site at the end of the putative V-like region of CD4. At the 3' end of the coding region, a translational stop codon is added as well as a sequence which allows this end to be ligated to BamHl restriction fragments. The synthetic DNA is made in 8 fragments, 4 for each strand, 70-90 bases long. These are then allowed to anneal and ligated prior to isolation on a polyacrylamide gel (fragment 33).

pRKCD4 is digested with EcoRI and BspMl and the 478bp fragment containing the region coding for the putative V-like domain of CD4 is recovered (fragment 34). Fragments 33 and 34 are ligated together with fragment 16 (from the expression vector pRK5). The ligation mixture is transformed into E. coli strain 294, the transformed culture plated on ampicillin media plates and resistant colonies selected. Plasmid DNA is prepared from transformants and checked by restriction analysis for the presence of the correct fragment. The resulting plasmid is referred to as pRKCD4Ck.

A plasmid encoding a fusion of the CD4 V-like domain to the human immunoglobulin Cγ2 region is constructed in a similar fashion, and is referred to as pRKCD4Cγ2. Both of these plasmids are transfected into 293 cells, myeloma cells or other competent cells in order to obtain cell lines expressing variant CD4 molecules as described above.

### Expression in CHO Cells

Plasmids were constructed to direct the expression of the immunoadhesons described above in CHO cells. These are referred to as pSVeCD4_{4γ1}SVDHFR, pSVeCD4_{2γ1}SVDHFR, pSVeCD4_{e4γ1}SVDHFR, pSVeCD4_{e2γ1}SVDHFR, pSVeCD4_{4κ}SVDHFR and pSVeCD4_{2κ}SVDHFR.

Fragment 31 was prepared as described above. Fragment 32a was prepared by digesting plasmid pE348HBV. E400 D22 with BamHl, blunting with Klenow fragment and the four dNTPs, then digesting with Pvul. Plasmids pRKCD4_{4γ1}, pRKCD4_{2γ1}, pRKCD4_{e4γ1}, pRKCD4_{e2γ1}, pRKCD4_{4κ} and pRKCD4_{2κ} were separately digested with Hindlll, blunted with Klenow fragment and the four dNTPs, then digested with *EcoRI*. The resulting DNA fragments were ligated together with fragments 31 and 32a and transformed into *E. coli* strain 294. Colonies were selected and checked for the presence of the correct plasmid as above, then transfected into CHO cells and amplified by methotrexate selection using conventional procedures.

### Example 3

The gDCD4T secreted by the method of Example 1 was purified from cell culture fluid containing either 10% FBS (fetal bovine serum) or no added FBS. The conditioned cell culture fluid was first concentrated by ultrafiltration then purified by immunoaffinity chromatography. The immunoaffinity column was produced by coupling murine monoclonal antibody 5B6 (whose epitope is on the HSV-1 gD portion of the gDCD4T molecule) to glyceryl coated controlled pore glass by the method of Roy et al. 1984. The concentrated cell culture fluid is applied directly to the column and the contaminating proteins are washed away with neutral pH buffer. The column is then washed with neutral buffer containing tetramethylammonium chloride followed by neutral buffer containing Tween 80. The bound gDCD4T is eluted from the column with buffer at pH3 containing Tween 80 (0.1% w/v) and is neutralized immediately as it is eluted. The eluted neutralized gDCD4T is then concentrated by ultrafiltration and dialyzed/diafiltered to exchange the buffer for a physiological salt solution containing Tween 80 at approximately 0.1% w/v.

If the detergent is not present the gDCD4T forms aggregates as evidenced by the ability of centrifugation at approximately 10,000 Xg for 2 minutes to remove the gDCD4T from the solution. Incubation of gDCD4T at 4°C in 0.1M sodium acetate, 0.5M NaCl and 0.25M tris at pH 7 together with BSA, Tween 80 or glycerol as candidate stabilizers showed that, in the absence of a stabilizer the gDCD4T gradually aggregated over the space of 12 days to the point where only about 60-70% of the protein was soluble. However, use of 0.1% w/v Tween 80 or (0.5 mg/ml BSA ensured that about 100% or 80%, respectively, of the gDCD4T remained soluble over this period. Surprisingly glycerol was ineffective as a stabilizer and produced results inferior even to the control-at 8 days about 80% of the gDCD4T was aggregated when stored in the presence of glycerol.

### Example 4

Plasmids were constructed to direct the expression of proteins containing differing lengths of the amino-terminal, extracellular domain of CD4 fused to the constant region of human immunoglobulin γ1. These plasmids are referred to as pRKCD4_{2γ1}, pRKCD4_{e4γ1}, pRKCD4_{2γ1}, pRKCD4_{e2γ1}, pRKCD4_{1γ1}, and pRKCD4_{e1γ1}.

Plasmid pRKCD4_{4γ1} contains the portion of the CD4 gene from the initiation codon to the fusion site after the codon for serine reside 366 of the mature CD4 polypeptide, immediately followed by the sequence coding for the constant region of human immunoglobulin γ1, starting at the codon for serine residue 114 of mature human immunoglobulin γ1 (Kabat *et al.*).

Plasmid pRKCD4_{e4γ1} contains the portion of the CD4 gene from the initiation codon to the fusion site after the codon for lysine residue 360 of the mature CD4 polypeptide, immediately followed by the sequence coding for the constant region of human immunoglobulin γ1, starting at the codon for serine residue 114 of mature human immunoglobulin γ1 (Kabat *et al.*).

Plasmid pRKCD4_{2γ1} contains the portion of the CD4 gene from the initiation codon to the fusion site after the codon for glutamine residue 180 of the mature CD4 polypeptide, immediately followed by the sequence coding for the constant region of human immunoglobulin γ1, starting at the codon for serine residue 114 of mature human immunoglobulin γ1 (Kabat *et al*.).

Plasmid pRKCD4_{e2γ1} contains the portion of the CD4 gene from the initiation codon to the fusion site after the codon for leucine residue 177 of the mature CD4 polypeptide, immediately followed by the sequence coding for the constant region of human immunoglobulin γ1, starting at the codon for serine residue 114 of mature human immunoglobulin γ1 (Kabat *et al.*).

Plasmid pRKCD4_{1γ1} contains the portion of the CD4 gene from the initiation codon to the fusion site after the codon for aspartic acid residue 105 of the mature CD4 polypeptide, immediately followed by the sequence coding for the constant region of human immunoglobulin γ1, starting at the codon for serine residue 114 of mature immunoglobulin γ1 (Kabat et al.).

Plasmid pRKCD4_{e1γ1} contains the portion of the CD4 gene from the initiation codon to the fusion site after the codon for leucine residue 100 of the mature CD4 polypeptide, immediately followed by the sequence coding for the constant region of human immunoglobulin γ1, starting at the codon for serine residue 114 of mature human immunoglobulin γ1 (Kabat *et al*.).

Construction of these plasmids required the prior construction of plasmid pRKCD4TP/γ1. It was constructed as follows:

A cDNA clone coding for human immunoglobulin γ1 was obtained from a human spleen cDNA library (Clontech Laboratories, Inc.) using oligonucleotides based on the published sequence (Ellison *et al.*, "Nucl. Acids Res." 10: 4071-4079 [1982]), and an EcoRI-Eagi fragment (the EcoRI site was contributed by a linker) containing part of the variable and all of the constant region was obtained. This fragment was blunted with Klenow fragment, and recovered by gel electrophoresis (Fragment al).

Plasmid pRKCD4TP was digested with Xbal and treated with Klenow Enzyme, and Fragment a2, containing the linearized plasmid was recovered by gel electrophoresis, and ligated with fragment al. The ligation mixture was transformed into E. coli strain 294, the transformed culture plated on ampicillin media plates and resistant colonies selected. Plasmid DNA was prepared from the transformants and checked by restriction analysis for the presence of the correct fragment in the correct orientation (i.e., the immunoglobulin coding region in the same orientation as the CD4 coding region, and at the 3' end of the CD4 coding region). This plasmid is referred to as pRKCD4TP/γ1.

Synthetic oligonucleotides were made as primers for deletional mutagenesis reactions to fuse the appropriate coding sequences of IgG1 and CD4 as described above. These were synthesized as 48-mers comprising 24 nucleotides on each side of the desired fusion site (i.e., corresponding to the COOH-terminal 8 residues of the desired CD4 moiety, and the NH₂-terminal 8 residues of the desired immunoglobulin moiety). Plasmid pRKCD4TP/γ1 was transformed into E. coli strain SR101 and the transformed cultures plated on ampicillin media plates. Resistant colonies were selected and grown in the presence of m13KO7 bacteriophage to yield secreted, encapsidated single-stranded templates of pRKCD4TP/γ1. The single-stranded plasmid DNA was isolated and used as the template for mutagenesis reactions with the synthetic oligonucleotides described above as primers. The mutagenesis reactions were transformed E. coli SR101 and the transformed culture plated on ampicillin media plates. Transformants were screened by colony hybridization (ref. Grunstein-Hogness) for the presence of the appropriate fusion site, using 16mers as probes. These 16mers comprise 8 bases on either side of the fusion site, and the hybridization conditions chosen were sufficiently stringent that the probes only detect the correctly fused product. Colonies identified as positive were selected and plasmid DNA was isolated and transformed into E. coli strain SR101. The transformed cultures were plated on ampicillin media plates, and resistant colonies were selected and grown in the presence of m13KO7 bacteriophage. Templates were prepared as above and screened by sequencing.

The plasmids were transfected into 293 cells using standard procedures and assayed for expression and production as described above.

| | Expressed | Secreted |
|---|---|---|
| pRKCD4_{e1γ}1 | - | - |
| pRKCD4_{1γ1} | + | - |
| pRKCD4_{e2γ1} | + | + |
| pRKCD4_{2γ1} | + | + |
| pRKCD4_{e4γ1} | + | + |
| pRKCD4_{4γ1} | + | + |

Plasmids also were constructed to direct the expression of fusion proteins containing differing lengths of the amino-terminal, extracellular domain of CD4 fused to the truncated portion of the constant region of human immunoglobulin γ1, comprising only the hinge region and constant domains CH₂ and CH₃.

Synthetic oligonucleotides were made as primers for mutagenesis reactions to delete the immunoglobulin sequence from Ser114 to Cys215 inclusive (Kabat *et al*.). These were synthesized as 48-mers comprising 242γ nucleotides on each side of the desired fusion site (i.e., corresponding to the COOH-terminal 8 residues of the desired CD4 moiety, and the NH₂-terminal 8 residues of the desired immunoglobulin moiety). Plasmids pRKCD4_{4γ1}, pRKCD4_{2γ1} and pRKCD4_{1γ1} were separately transformed into E. coli strain SR101 and the transformed culture plated on ampicillin media plates. Transformants were screened by colony hybridization (Grunstein-Hogness) for the presence of the appropriate fusion site, using 16mers as probes. These 16mers comprise 8 bases on either side of the fusion site, and the hybridization conditions chosen were sufficiently stringent that the probes only detect the correctly fused product. Colonies identified as positive were selected and plasmid DNA was isolated and transformed into E. coli strain SR101. The transformed cultures were plated on ampicillin media plates, and resistant colonies were selected and grown in the presence of m13KO7 bacteriophage. Templates were prepared as above and screened by sequencing.

The plasmid derived from plasmid pRKCD4_{4γ1} is referred to as pRKCD4_{4Fc1}, that derived from plasmid pRKCD4_{2γ1} is referred to as pRKCD4_{2Fc1} and that derived from plasmid pRKCD4_{1γ1} is referred to as pRKCD4_{1Fc1}.

pRKCD4_{2Fc1}, pRKCD4_{1Fc1} and pRKCD4_{4Fc1} are cultured in the same fashion as described above and CH1-deleted CD4 immunoadhesons recovered as described elsewhere herein.

### Light Chain Fusions

Plasmids were constructed to direct the expression of proteins containing differing lengths of the amino terminal, extracellular domain of CD4 fused to the constant region of human immunoglobulin κ. These plasmids are referred to as pRKCD4_{4κ}, and pRKCD4_{e4κ}.

Plasmid pRKCD4_{4κ} contains the portion of the CD4 gene from the initiation codon to the fusion site after the codon for serine residue 366 of the mature CD4 polypeptide, immediately followed by the sequence for the constant region of human immunoglobulin κ, starting at the codon for threonine residue 109 of the mature human immunoglobulin κ. (Kabat *at al*.).

Plasmid pRKCD4_{e4κ} contains the portion of the CD4 gene from the initiation codon to the fusion site after the codon for lysine residue 360 of the mature CD4 polypeptide, immediately followed by the sequence for the constant region of human immunoglobulin κ, starting at the codon forthreonine residue 109 of the mature human immunoglobulin κ (Kabat *et al*.)

These plasmids were constructed in a manner analogous to plasmid pRKCD4_{γ1} described above, with the following exception:

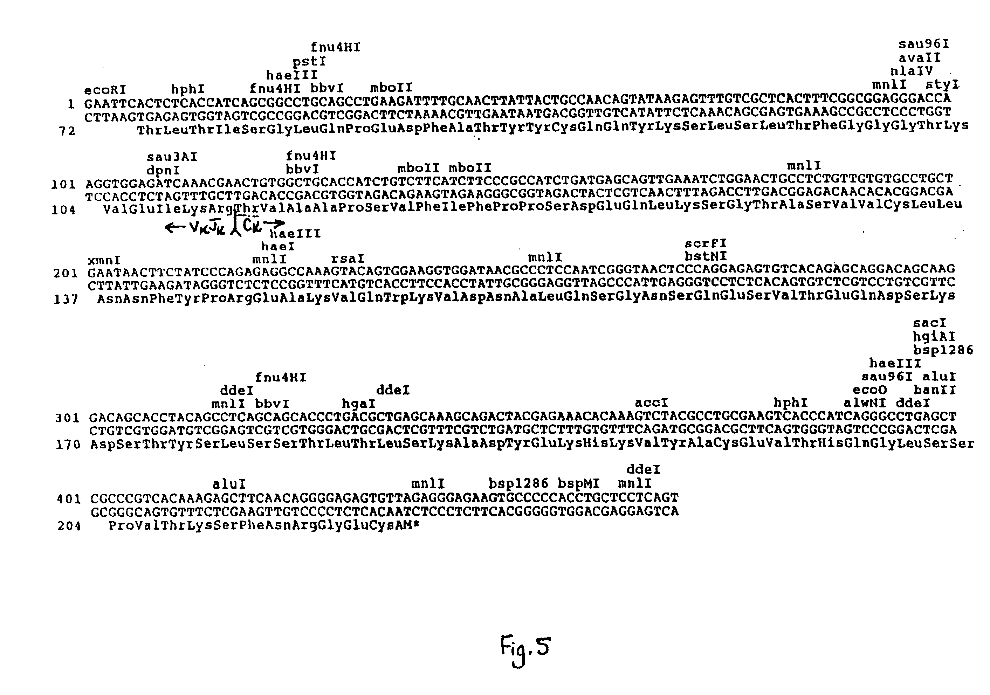

The human immunoglobulin κ coding sequence (Fig. 5) was obtainedfrom ahuman spleen cDNA library (Clontech Laboratories, Inc.) using oligonucleotides based on the published sequence (Hieter, P.A. *et al*., Cell 22:197-207 [1980]) and an *EcoRI*-BspMI fragment containing part of the variable region and the entire constant region was obtained. This fragment was blunted with Klenow fragment and the four dNTPs. This fragment was used instead of fragment al, and was used to construct plasmid pRKCD4TP/hκ.

### Example 5

### Culture, Purification and formulation of CD4 variants

Plasmids encoding CD4T, prolyl terminal (CD4TP), or CD4T immunoadhesons were calcium phosphate transfected into CHO-DP7 (a proinsulin-transformed autocrine host cell derived from CHO; U.S.S.N. 97,472) and the transformants grown in selective medium (1:1 HAM F12/DMEM GHT⁻ containing 1 - 10% diafiltered or dialyzed bovine serum). Other suitable host cells are CHO cells or 293s human embryonic kidney cells. The transformants were subcloned into the same medium but containing 500 nm methotrexate. A subclone capable of secreting CD4T, CD4tp 500 b, was selected. CD4tp 500 b is cultured in a DMEM/HAM F12 medium at about 37°C until CD4T accumulates in the culture, after which the medium is separated from the cells and insoluble matter by centrifuging.

Culture fluid from CD4TP transformants was concentrated and diafiltered to lower the ionic strength. The concentrate was passed through a large volume of Q-Sepharose anion exchange resin (previously equilibrated with 25 mM NaCl, pH 8.5) in order to adsorb contaminants from the culture fluid. The isoelectric point of CD4TP is about 9.5, thus making it possible to discriminate between truncated forms of CD4 and most contaminants by alternate adsorption, respectively, on a cation exchange resin such as carboxymethyl or sulfonyl Sepharose, and an anion exchange resin such a quaternary ammonium Sepharose. In addition, since highly electropositive domains are present in the extracellular segment of CD4 any CD4-containing variant is purified in the same fashion as CD4TP. The unadsorbed culture fluid from the anion exchange resin step was then passed through a cation exchange resin (previously equilibrated with 25 mM NaCl at pH 8.5) whereby CD4TP was adsorbed to the resin. The CD4TP was eluted with a NaCl gradient at pH 8.5, this CD variant eluting at about 0.2 mM NaCl. Ammonium sulfate was added to the eluate to the concentration of 1.7M and the solution passed through a column of hydrophobic interaction chromatography resin (phenyl or butyl Sepharose). The CD4TP was eluted from the hydrophobic interaction column with a gradient of ammonium sulfate, the CD4TP emerging at about 0.7M ammonium sulfate. The eluate was concentrated and buffer exchanged on a G-25 column using phosphate buffered saline containing .02 % (w/v) Tween 20 or Tween 80. The CD4TP was soluble and stable in this solution, which was sterile filtered and filled into vials as an aqueous formulation. Other polymeric nonionic surfactants are suitably used with the CD4 formulations, including Pluronic block copolymers or polyethylene glycol.

It is also possible to employ immunoaffinity purification of soluble CD4 wherein the CD4 is adsorbed onto an immobilized antibody against CD4. This method suffers from the disadvantage that elution of the CD4T under acidic conditions leads to protein aggregation that is only thoroughly ameliorated at relatively higher levels of surfactant. The foregoing procedure permits the use of much lower quantities of surfactant, about from 0.01 to 0.10 % (w/v) surfactant.

The procedure followed for the purification of CD4 fusions with immunoglobulin heavy chain was to concentrate recombinant supernatants by ultrafiltration and thereafter adsorb the fusion onto resin-immobilized Staphylococcal protein A. The fusion was eluted with 0.1M citrate buffer pH 3 with no salt or detergent. This preparation is buffered into tris buffer at pH 7.5. The immunoglobulin fusions with CD4 V1-V4 optionally are further purified by the procedure described above for unfused CD4 variants. CD4 immunoglobulin fusions with CD4 V1-V2 also may be purified by the procedure above, except that it is not expected that the isoelectric point of this class of molecules will be as alkaline as that of species containing all four V regions of CD4.

### Example 6

The characteristics of several adheson variants were determined. As shown in table 4 the immunoadhesons CD4_{4γ1} and CD4_{2γ1} show improved plasma half-life in rabbits, coupled with high-affinity gp120 binding and an affinity for FCγ receptor (determined with U937 cells) that is comparable to that of bulk human IgG.

**Table 4**

| | gp120 KD (nM)^{#} | FcγR KD (nM)⁺ | Plasma Half-Life⁺⁺ In Rabbits (Hrs.) |
|---|---|---|---|
| CD4T^{§} | 2.3 ± 0.4 | - | 0.25 |
| CD4_{4γ1} | 1.2 ± 0.1 | 2.83 ± 0.25 | 6.4 |
| CD4_{2γ1} | 1.4 ± 0.1 | 3.01 ± 0.68 | 40.6 |
| human IgG | ND | 3.52 ± 0.5 | 21 days* |

| | | | |
|---|---|---|---|
| * determined in humans | | | |
| ⁺ KD was determined by the method of Anderson *et al*., "J. Immunol." 125:2735-2741 (1980). | | | |
| ^{#} determined by the method of Smith *et al*., "Science" 238:1704-07 (1987). | | | |
| § residues 1-368 only | | | |
| ++ The adheson variant was injected intravenously into rabbits and samples of blood were collected periodically and assayed for the presence of the adheson variant. | | | |

## Claims

1. A process which comprises makingan immunoglobulin heavy chain dimer in which the extracellular domain sequence of a cell surface member of the immunoglobulin gene superfamily, which superfamily member is not a class I or class II major histocompatibility antigen, an immunoglobulin, nor a T-cell receptor α, β, γ or δ chain, is substituted for the variable region of an immunoglobulin heavy chain, which dimer comprises a polypeptide comprising said extracellular domain fused to an immunoglobulin heavy chain constant region, and in which dimer said extracellular domain binds a ligand of said member of the immunoglobulin gene superfamily.

2. A process according to claim 1 wherein the immunoglobulin heavy chain dimer is divalent for said ligand.

3. The process of claim 1 or claim 2 wherein the immunoglobulin sequence is obtained from IgG1, IgG2, IgG3, IgG4, IgA, IgE, IgD or IgM.

4. The process of any preceding claim wherein the superfamily member is CD1, CD2, CD3, CD4, CD8, CD28, OX-2, Thy-1, an intercellular or neural adhesion molecule, poly-Ig receptor, myelin associated glycoprotein, high affinity IgE receptor, platelet derived growth factor receptor, colony stimulating factor-1 receptor, Fc gamma receptors or the carcinoembryonic antigen.

5. The process of claim 4 wherein the superfamily member is CD2, CD4, CD8, CD28, the γ, δ or ε chain of CD3 or the high affinity IgE receptor.

6. The process of claim 5 wherein the superfamily member is CD4.

7. The process of claim 1 wherein the V₁ domain of CD4 or CD4 lacking the transmembrane and cytoplasmic domains is fused at its C-terminus to said constant region.

8. A process which comprises making a polypeptide comprising an extracellular domain of CD4 fused to a polypeptide extraneous to CD4.

9. The process of claim 8 wherein the extraneous polypeptide comprises a signal sequence.

10. The process of claim 8 or claim 9 wherein the extraneous polypeptide is capable of eliciting a humoral immune response in an animal.

11. The process of claim 10 wherein the extraneous polypeptide is a viral polypeptide or an allergen.

12. The process of claim 8 wherein the extraneous polypeptide is albumin, apolipoprotein or transferrin.

13. The process of claim 8 wherein the extraneous polypeptide is a cytotoxic polypeptide.

14. A process which comprises making a CD4 polypeptide amino acid sequence variant (a) having a transmembrane domain modified to be incapable of becoming lodged in the cell membrane, or (b) lacking a transmembrane domain.

15. A process according to claim 14 wherein the transmembrane domain has been modified by substituting an amino acid sequence having a substantially hydrophilic hydropathy profile.

16. A process which comprises making a composition comprising a CD4 polypeptide amino acid sequence variant (a) having a transmembrane domain modified to be incapable of cell membrane anchorage, or (b) lacking a transmembrane domain.

17. The process of claim 16 wherein the variant comprises a CD4 amino acid sequence capable of binding gp120.

18. A process which comprises making a CD4 polypeptide variant comprising a fusion protein in which a polypeptide comprising a human CD4 antigen variable (V) region is fused at its C-terminus to the N-terminus of a polypeptide comprising a constant region of an immunoglobulin chain covalently associated with a companion immunoglobulin heavy chain-light chain pair bearing a V_{L}V_{H} antibody combining site capable of binding to a predetermined antigen.

19. The process of claim 18 wherein the CD4 polypeptide variant is AC_{H}-(V_{L}C_{L}-V_{H}C_{H}), (AC_{L}-AC_{H})-(V_{L}C_{L}-V_{H}C_{H}), (AC_{L}-V_{H}C_{H})-(V_{L}C_{L}-V_{H}C_{H}), or (V_{L}C_{L}-AC_{H})-(V_{L}C_{L}-V_{H}C_{H}), wherein A is a human CD4 antigen V region, V_{L} and V_{H} are the variable domains of an immunoglobulin light and heavy chain, respectively, and C_{L} and C_{H} are the constant domains of an Immunoglobulin light and heavy chain, respectively.

20. The process of claim 18 or claim 19 wherein the fusion protein comprises the V-J domain of the human CD4 antigen.

21. The process of claim 18 or claim 19 wherein the human CD4 antigen comprises the sequence Asn-Lys-Val-Val-Leu-Gly-Lys-Lys.

22. The process of any one of claims 18 to 21 wherein the immunoglobulin sequences are from IgG1, IgG2, IgG3, IgG4, IgA, IgE, IgD or IgM.

23. The process of claim 22 wherein the immunoglobulin sequences are from IgG-1 or IgG-3.

24. The process of claim 22 wherein the immunoglobulin sequences are from IgA or IgM.

25. A process which comprises making nucleic acid encoding a dimer according to any one of claims 1 to 7.

26. A process which comprises making replicable vector comprising nucleic acid prepared by the process of claim 25.

27. A recombinant host cell comprising nucleic acid prepared by the process of claim 25 or the recombinant vector prepared by the process of claim 26.

28. A method for preparing a dimer according to any one of claims 1 to 7 comprising recovering the dimer from the culture of a recombinant host cell according to claim 27.

29. A process which comprises making nucleic acid encoding a polypeptide according to any one of claims 8 to 13.

30. A process which comprises making a replicable vector comprising nucleic acid prepared by the process of claim 29.

31. A recombinant host cell comprising nucleic acid prepared by the process of claim 29 or the recombinant vector prepared by the process of claim 30.

32. A method for preparing a polypeptide according to any one of claims 8 to 13 comprising recovering the polypeptide from the culture of a recombinant host cell according to claim 31.

33. A process which comprises making nucleic acid encoding a polypeptide according to claim 14 or claim 15.

34. A method for preparing a CD4 polypeptide amino acid sequence variant according to claim 14 or claim 15 comprising recovering the variant from the culture of a host cell transfected with the nucleic acid prepared by the process of claim 37.

35. A process which comprises making nucleic acid encoding a CD4 polypeptide variant according to any one of claims 18 to 25.

36. A method of preparing a CD4 polypeptide variant comprising transfecting a host cell expressing nucleic acid encoding an immunoglobulin having a variable region directed against a predetermined antigen, with nucleic acid encoding a fusion protein in which a polypeptide comprising a human CD4 antigen variable (V) region is fused at its C-terminus to the N-terminus of a polypeptide comprising a constant region of an immunoglobulin chain.

37. The method of claim 36 wherein the CD4 polypeptide variant is recovered from the host cell culture.

## Patentansprüche

1. Verfahren, umfassend die Herstellung eines Immunglobulin-Schwerketten-Dimers, worin der variable Bereich einer Immunglobulin-Schwerkette durch die Sequenz der extrazellulären Domäne eines Zelloberfiächenmitglieds der Immunglobulin-Gen-Überfamilie ersetzt ist, wobei das Überfamilienmitglied kein Haupt-Histokompatibilitäts-Antigen der Klasse I oder Klasse II, kein Immunglobulin und weder die α-, β-, γ- noch die δ-Kette eines T-Zellenrezeptors ist, wobei das Dimer ein Polypeptid umfaßt, das die extrazelluläre Domäne mit einem konstanten Immunglobulin-Schwerketten-Bereich fusioniert umfaßt, und wobei in diesem Dimer die extrazelluläre Domäne einen Liganden des Mitglieds der Immunglobulin-Gen-Überfamilie bindet.

2. Verfahren nach Anspruch 1, worin das Immunglobulin-Schwerketten-Dimer für diesen Liganden zweiwertig ist.

3. Verfahren nach Anspruch 1 oder Anspruch 2, worin die Immunglobulinsequenz von IgG1, IgG2, IgG3, IgG4, IgA, IgE, IgD oder IgM erhalten wird.

4. Verfahren nach einem der vorangegangenen Ansprüche, worin das Überfamilienmitglied CD1, CD2, CD3, CD4, CD8, CD28, OX-2, Thy-1, ein interzelluläres oder neurales Bindemolekül, Poly-Ig-Rezeptor, mit Myelin assoziiertes Glykoprotein, IgE-Rezeptor mit hoher Affinität, von Blutplättchen stammender Wachstumsfaktorrezeptor, Kolonie stimulierender Faktor-1-Rezeptor, Fc-γ-Rezeptoren oder das carcinoembryonale Antigen ist.

5. Verfahren nach Anspruch 4, worin das Überfamilienmitglied CD2, CD4, CD8, CD28, die γ-, δ- oder ε-Kette von CD3 oder der IgE-Rezeptor mit hoher Affinität ist.

6. Verfahren nach Anspruch 5, worin das Überfamilienmitglied CD4 ist.

7. Verfahren nach Anspruch 1, worin die V₁-Domäne von CD4 oder CD4 ohne Transmembran- und zytoplasmatische Domänen an ihrem C-Terminus mit dem konstanten Bereich fusioniert ist.

8. Verfahren, umfassend die Herstellung eines Polypeptids, das eine extrazellulare Domäne von CD4, fusioniert mit einem zu CD4 fremden Polypeptid, umfaßt.

9. Verfahren nach Anspruch 8, worin das fremde Polypeptid eine Signalsequenz umfaßt.

10. Verfahren nach Anspruch 8 oder 9, worin das fremde Polypeptid bei einem Tier eine humorale Immunreaktion hervorrufen kann.

11. Verfahren nach Anspruch 10, worin das fremde Polypeptid ein virales Polypeptid oder ein Allergen ist.

12. Verfahren nach Anspruch 8, worin das fremde Polypeptid Albumin, Apolipoprotein oder Transferrin ist.

13. Verfahren nach Anspruch 8, worin das fremde Polypeptid ein zytotoxisches Polypeptid ist.

14. Verfahren, umfassend die Herstellung einer CD4-Polypeptid-Aminosäuresequenzvariante, bei der (a) eine Transmembrandomäne so modifiziert ist, daß sie nicht mehr fähig ist, sich in der Zellmembran anzuordnen, oder der (b) eine Transmembrandomäne fehlt.

15. Verfahren nach Anspruch 14, worin die Transmembrandomäne durch Substitution einer Aminosäuresequenz mit einem im wesentlichen hydrophilen Hydropathieprofil modifiziert worden ist.

16. Verfahren, umfassend die Herstellung einer Zusammensetzung, die eine CD4-Polypeptid-Aminosäuresequenzvariante umfaßt, welche (a) eine Transmembrandomäne aufweist, die so modifiziert ist, daß sie nicht mehr zur Verankerung in der Zellmembran fähig ist, oder der (b) eine Transmembrandomäne fehlt.

17. Verfahren nach Anspruch 16, worin die Variante eine CD4-Aminosäuresequenz umfaßt, die zur Bindung von gp120 fähig ist.

18. Verfahren zur Herstellung einer CD4-Polypeptidvariante, die, ein Fusionsprotein umfaßt, in dem ein Polypeptid, das einen variablen (V-) menschlichen CD4-Antigen-Bereich umfaßt, an seinem C-Terminus mit dem N-Terminus eines Polypeptids fusioniert ist, das einen konstanten Bereich einer Immunglobulinkette umfaßt, die kovalent mit einem begleitenden Immunglobulin-Schwerketten-Leichtketten-Paar assoziiert ist, das eine V_{L}V_{H}-Antikörperkombinationsstelle umfaßt, die zur Bindung an ein vorbestimmtes Antigen fähig ist.

19. Verfahren nach Anspruch 18, worin die CD4-Polypeptidvariante AC_{H}-(V_{L}C_{L}-V_{H}C_{H}), (AC_{L}-AC_{H})-(V_{L}C_{L}-V_{H}C_{H}), (AC_{L}-V_{H}C_{H})-(V_{L}C_{L}-V_{H}C_{H}) oder (V_{L}C_{L}-AC_{H})-(V_{L}C_{L}-V_{H}C_{H}) ist, worin A ein menschlicher CD4-Antigen-V-Bereich ist, V_{L} und V_{H} die variablen Domänen der leichten bzw. schweren Kette eines Immunglobulins sind und C_{L} und C_{H} die konstanten Domänen der leichten bzw. schweren Kette eines Immunglobulins sind.

20. Verfahren nach Anspruch 18 oder 19, worin das Fusionsprotein die V-J-Domäne des menschlichen CD4-Antigens umfaßt.

21. Verfahren nach Anspruch 18 oder 19, worin das menschliche CD4-Antigen die Sequenz Asn-Lys-Val-Val-Leu-Gly-Lys-Lys umfaßt.

22. Verfahren nach einem der Ansprüche 18 bis 21, worin die Immunglobulinsequenzen von IgG1, IgG2, IgG3, IgG4, IgA, IgE, IgD oder IgM stammen.

23. Verfahren nach Anspruch 22, worin die Immunglobulinsequenzen von IgG-1 oder IgG-3 stammen.

24. Verfahren nach Anspruch 22, worin die Immunglobulinsequenzen von IgA oder IgM stammen.

25. Verfahren zur Herstellung von Nukleinsäure, die für ein Dimer nach einem der Ansprüche 1 bis 7 kodiert.

26. Verfahren, umfassend die Herstellung eines replizierbaren Vektors, der die nach dem Verfahren nach Anspruch 25 hergestellte Nukleinsäure umfaßt.

27. Rekombinante Wirtszelle, umfassend die nach dem Verfahren nach Anspruch 25 hergestellte Nukleinsäure oder den nach dem Verfahren nach Anspruch 26 hergestellten rekombinanten Vektor.

28. Verfahren zur Herstellung eines Dimers nach einem der Ansprüche 1 bis 7, umfassend die Gewinnung des Dimers aus der Kultur einer rekombinanten Wirtszelle nach Anspruch 27.

29. Verfahren, umfassend die Herstellung von Nukleinsäure, die für ein Polypeptid nach einem der Ansprüche 8 bis 13 kodiert.

30. Verfahren, umfassend die Herstellung eines replizierbaren Vektors, der die nach dem Verfahren nach Anspruch 29 hergestellte Nukleinsäure umfaßt.

31. Rekombinante Wirtszelle, die die nach dem Verfahren nach Anspruch 29 hergestellte Nukleinsäure oder den nach dem Verfahren nach Anspruch 30 hergestellten rekombinanten Vektor umfaßt.

32. Verfahren zur Herstellung eines Polypeptids nach einem der Ansprüche 8 bis 13, umfassend die Gewinnung des Polypeptids aus der Kultur einer rekombinanten Wirtszelle nach Anspruch 31.

33. Verfahren, umfassend die Herstellung von Nukleinsäure, die für ein Polypeptid nach Anspruch 14 oder 15 kodiert.

34. Verfahren zur Herstellung einer CD4-Polypeptid-Aminosäuresequenzvariante nach Anspruch 14 oder 15, umfassend das Gewinnen der Variante aus der Kultur einer Wirtszelle, die mit der nach dem Verfahren nach Anspruch 33 hergestellten Nukleinsäure transfiziert ist.

35. Verfahren, umfassend die Herstellung von Nukleinsäure, die für eine CD4-Polypeptid-Variante nach einem der Ansprüche 18 bis 25 kodiert.

36. Verfahren zur Herstellung einer CD4-Polypeptidvariante, umfassend das Transfizieren einer Wirtszelle, die Nukleinsäure exprimiert, welche für ein Immunglobulin mit einem variablen Bereich kodiert, der gegen ein vorbestimmtes Antigen gerichtet ist, mit Nukleinsäure, die für ein Fusionsprotein kodiert, in dem ein Polypeptid, das einen variablen (V-) menschlichen CD4-Antigen-Bereich umfaßt, an seinem C-Terminus mit dem N-Terminus eines Polypeptids fusioniert ist, das einen konstanten Bereich einer Immunglobulinkette umfaßt.

37. Verfahren nach Anspruch 36, worin die CD4-Polypeptid-Variante aus der Wirtszellenkultur gewonnen wird.

## Revendications

1. Procédé qui comprend la fabrication d'un dimère chaîne lourde d'immunoglobuline dans lequel la séquence du domaine extracellulaire d'un membre de surface de cellule de la superfamille du gène de l'immunoglobuline, lequel membre de la superf amille n'est pas un antigène d'histocompatibilité majeur de classe I ou de classe II, une immunoglobuline ni une chaîne α, β, γ ou δ d'un récepteur de cellules T, est substituée pour la région variable d'une chaîne lourde d'immunoglobuline, lequel dimère comprend un polypeptide comprenant ledit domaine extracellulaire fusionné à une région constante de chaîne lourde d'immunoglobuline dimère dans lequel ledit domaine extracellulaire lie un ligand dudit membre de la superfamille du gène d'immunoglobuline.

2. Procédé selon la revendication 1, où le dimère chaîne lourde d'immunoglobuline est divalent pour ledit ligand.

3. Procédé selon la revendication 1 ou 2 où la séquence d'immunoglobuline est obtenue à partir de IgG1, IgG2, IgG3, IgG4, IgA, IgE, IgD ou IgM.

4. Procédé selon l'une quelconque des revendications précédentes dans lequel le membre de la superfamille est CD1, CD2, CD3, CD4, CD8, CD28, OX -2, Thy-1, une molécule d'adhésion neurale ou intracellulaire, un récepteur poly-Ig, une glycoprotéine associée à une myéline, un récepteur d'IgE à affinité élevée, un récepteur d'un facteur de croissance dérivé des plaquettes, un récepteur du facteur-1 stimulant les colonies, les récepteurs gamma de Fc ou l'antigène carcinoembryonnaire.

5. Procédé selon la revendication 4 où le membre de la superfamille est CD2, CD4, CD8, CD28, la chaîne γ, δ ou ε de CD3 ou le récepteur d'IgE à affinité élevée.

6. Procédé selon la revendication 5 où le membre de la superfamille est CD4.

7. Procédé selon la revendication 1 où le domaine V₁ de CD4 ou CD4 manquant des domaines transmembrane et cytoplasmique est fusionné à son terminus C à ladite région constante.

8. Procédé qui comprend la fabrication d'un polypeptide comprenant un domaine extracellulaire de CD4 fusionné à un polypeptide étranger à CD4.

9. Procédé selon la revendication 8 où le polypeptide étranger comprend une séquence signal.

10. Procédé selon la revendication 8 ou la revendication 9 dans lequel le polypeptide étranger est capable d'élire une réponse immune humorale chez un animal.

11. Procédé selon la revendication 10 dans lequel le polypeptide étranger est un polypeptide viral ou un allergène.

12. Procédé selon la revendication 8 dans lequel le polypeptide étranger est l'albumine, une apolipoprotéine ou une transferrine.

13. Procédé selon la revendication 8 dans lequel le polypeptide étranger est un polypeptide cytotoxique.

14. Procédé qui comprend la fabrication d'un variant d'une séquence d'acides aminés du polypeptide CD4 (a) ayant un domaine transmembranaire modifié pour être incapable de venir se loger dans la membrane cellulaire, ou (b) manquant d'un domaine transmembranaire.

15. Procédé selon la revendication 14 dans lequel le domaine transmem branaire a été modifié par substitution d'une séquence d'acides aminés ayant un profil d'hydropathie substantiellement hydrophile.

16. Procédé qui comprend la fabrication d'une composition comprenant un variant d'une séquence d'acides aminés du polypeptide CD4 (a) ayant un domaine transmembranaire modifié pour être incapable d'un ancrage de cellules membranaires, ou (b) manquant d'un domaine transmembranaire.

17. Procédé selon la revendication 16 dans lequel le variant comprend une séquence d'acides aminés de CD4 capable de lier gp120.

18. Procédé qui comprend la fabrication d'un variant d'un polypeptide CD4 comprenant une protéine de fusion où un polypeptide comprenant une région variable (V) d'antigène de CD4 humain est fusionné à son terminus C au terminus N d'un polypeptide comprenant une région constante d'une chaîne d'immunoglobuline associée de façon covalente à une paire chaîne lourde-chaîne légère d'immunoglobuline compagnonne portant un site de combinaison d'anticorps V _{L}V_{H} capable de se lier à un antigène prédéterminé.

19. Procédé selon la revendication 18, dans lequel le polypeptide du variant de CD4 est AC _{H}-(V_{L}C_{L}-V_{H}C_{H}), (AC_{L}-AC_{H})-(V_{L}C_{L}-V_{H}C_{H}), (AC _{L}-V_{H}C_{H})-(V_{L}C_{L}-V_{H}C_{H}), ou (V _{L}C_{L}-AC_{H})-(V_{L}C_{L}-V_{H}C_{H}), où A est une région V d'antigêne de CD4 humain, V_{L} et V_{H} sont les domaines variables d'une chaîne légère et lourde d'immunoglobuline, respectivement, et C_{L} et C_{H} sont les domaines constants d'une chaîne légère et lourde d'immunoglobuline, respectivement.

20. Procédé selon la revendication 18 ou 19 où la protéine de fusion comprend le domaine V -J de l'antigène CD4 humain.

21. Procédé selon la revendication 18 ou la revendication 19 dans lequel l'antigène CD4 humain comprend la séquence Asn-Lys-Val-Val-Leu-Gly-Lys-Lys.

22. Procédé selon l'une quelconque des revendications 18 à 21 où les séquences d'immunoglobuline proviennent de IgG1, IgG2, IgG3, IgG4, IgA, IgE, IgD ou IgM.

23. Procédé selon la revendication 22 où les séquences d'immunoglobuline proviennent de IgG-1 ou IgG-3.

24. Procédé selon la revendication 22 dans lequel les séquences d'immunoglobuline proviennent de IgA ou IgM.

25. Procédé qui comprend la fabrication d'un acide nucléique encodant un dimère selon l'une quelconque des revendications 1 à 7.

26. Procédé qui comprend la fabrication d'un vecteur réplicable comprend un acide nucléique préparé par le procédé selon la revendication 25.

27. Cellule hôte recombinante comprenant un acide nucléique préparé par le procédé de la revendication 25 ou le vecteur recombinant préparé par le procédé de la revendication 26.

28. Méthode de préparation d'un dimère selon l'une que lconque des revendications 1 à 7 comprenant la récupération du polypeptide de la culture d'une cellule hôte recombinante selon la revendication 27.

29. Procédé qui comprend la fabrication d'un acide nucléique encodant un polypeptide selon l'une quelconque des revendications 8 à 13.

30. Procédé qui comprend la fabrication d'un vecteur réplicable comprenant un acide nucléique préparé par le procédé de la revendication 29.

31. Cellule hôte recombinante comprenant un acide nucléique préparé par le procédé de la revendication 29 ou le vecteur recombinant préparé par le procédé de la revendication 30.

32. Méthode de préparation d'un polypeptide selon l'une quelconque des revendications 8 à 13 comprenant la récupération du polypeptide de la culture d'une cellule hôte recombinante selon la revendication 31.

33. Procédé qui comprend la fabrication d'un acide nucléique encodant un polypeptide selon la revendication 14 ou la revendication 15.

34. Méthode pour préparer un variant d'une séquence d'acides aminés du polypeptide CD4 selon la revendication 14 ou la revendication 15 comprenant la récupération du variant de la culture d'une cellule hôte transfectée avec l'acide nucléique préparé par le procédé de la revendication 37.

35. Procédé qui comprend la fabrication d'un acide nucléique encodant un variant d'un polypeptide CD4 selon l'une quelconque des revendications 18 à 25.

36. Méthode de préparation d'un variant d'un polypeptide CD4 comprenant la transfectio n d'une cellule hôte exprimant un acide nucléique encodant une immunoglobuline ayant une région variable dirigée contre un antigène prédéterminé, avec l'acide nucléique encodant une protéine de fusion où un polypeptide comprenant une région variable (V) d' antigène de CD4 humain est fusionné à son terminus C au terminus N d'un polypeptide comprenant une région constante d'une chaîne d'immunoglobuline.

37. Méthode selon la revendication 36 dans laquelle le variant du polypeptide CD4 est récupéré de la culture de cellules hôtes.
